(19) Europäisches Patentamt
European Patent Office
Office européen des brevets

(11) Publication number: **0 084 408**
**A1**

# (12) EUROPEAN PATENT APPLICATION

(21) Application number: 83300030.0

(22) Date of filing: 05.01.83

(51) Int. Cl.³: C 07 C 59/46
C 07 C 69/732, C 07 C 177/00
A 61 K 31/215, A 61 K 31/19
A 61 K 31/557
//C07D317/72

(30) Priority: 12.01.82 JP 3765/82

(43) Date of publication of application:
27.07.83 Bulletin 83/30

(84) Designated Contracting States:
AT BE CH DE FR GB IT LI NL SE

(71) Applicant: SUMITOMO CHEMICAL COMPANY, LIMITED
15 Kitahama 5-chome Higashi-ku
Osaka-shi Osaka-fu(JP)

(72) Inventor: Sugie, Akihiko
10-1-116, Sonehigashinocho-2-chome
Toyonaka-shi(JP)

(72) Inventor: Ono, Keiichi
5-3-530, Higashiawaji-1-chome
Higashi-yodogawa-ku Osaka(JP)

(72) Inventor: Muraoka, Masami
14-7, Mefu-2-chome
Toakarazuka-shi(JP)

(72) Inventor: Yamamoto, Michihiro
16-40-309 Takagihigashimachi
Nishinomiya-shi(JP)

(74) Representative: Harrison, David Christopher et al,
MEWBURN ELLIS & CO 2/3 Cursitor Street
London EC4A 1BQ(GB)

## (54) Bicyclooctane compounds and their production and use.

(57) Novel bicyclooctane compounds of the general formula:

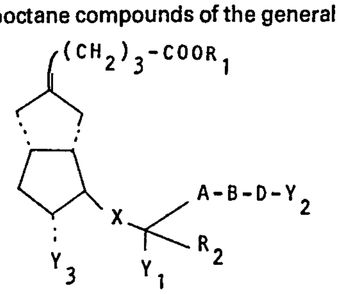

(I)

wherein

$R_1$ is a hydrogen atom or a $C_1$-$C_4$ alkyl group,
$R_2$ is a hydrogen atom or a $C_1$-$C_5$ alkyl group,
A is a $C_1$-$C_{10}$ alkylene group,
B is a direct bond, a cycloalkylene, or a group of one of the formulae: –O–, –CH=CH–, –C≡C–, 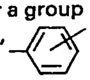
or -O- 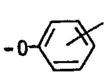 (o, m, p-phenylene or phenoxylene),
D is a $C_1$-$C_6$ alkylene or a cycloalkylene group,
X is an ethylene, a vinylene or an ethynylene group,
$Y_1$, $Y_2$ and $Y_3$ are each independently a free or a protected hydroxy group or a pharmaceutically acceptable salt of such a compound wherein R is a hydrogen atom and their pharmaceutically acceptable salts, and their production and use.

The compounds have potent platelet antiaggregation activity and hypotensive activity and are useful in treating and preventing thromboses and in treating hypertension, and the compounds have also potent antisecretory and antiulcerogenic activity and are useful in treating and preventing gastrointestinal ulcer.

EP 0 084 408 A1

# BICYCLOOCTANE COMPOUNDS AND THEIR PRODUCTION AND USE

The present invention relates to novel bicyclooctane compounds and to their production and use. More particularly, this invention relates to novel bicyclooctane compounds, to a pharmaceutical composition containing at least one of the bicyclooctane compounds and to a process for the production thereof.

The novel bicyclooctane compounds provided by the present invention are those represented by the formula [I]:

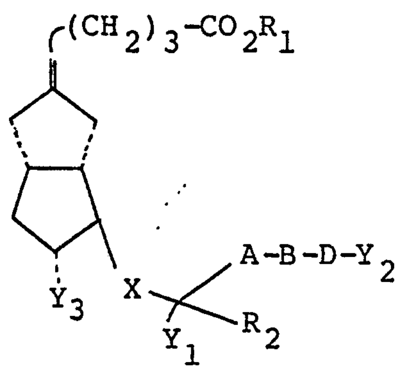

[I]

wherein $R_1$ is a hydrogen atom or a $C_1$-$C_4$ alkyl group, $R_2$ is a hydrogen atom or a $C_1$-$C_5$ alkyl group, A is a $C_1$-$C_{10}$ alkylene, B is a direct bond, a cycloalkylene or a group of ~~either~~ one of the formulae: -O-, -CH=CH-, -C≡C-, 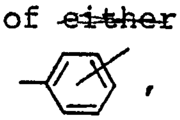, and -O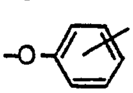 (o-, m-, p-phenylene or phenoxylene), D is a $C_1$-$C_5$ alkylene or a cycloalkylene, X is an ethylene group, a vinylene group or an ethynylene group and $Y_1$, $Y_2$ and $Y_3$ are each independently a free or a

protected hydroxy group, or a pharmaceutically acceptable salt of such a compound wherein $R_1$ is a hydrogen atom.

Among the bicyclooctane compounds of the formula [I], the preferred compounds are those in which $Y_1$, $Y_2$ and $Y_3$ are each free hydroxy group. Further more preferred compounds may be represented by the formula [II]:

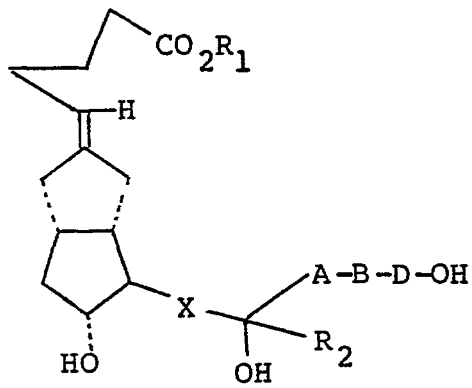

[II]

(E-form)

wherein $R_1$, $R_2$, A, B and D are each as defined above.

In the significances as used above, the term "$C_1$-$C_4$ alkyl" represented by $R_1$ means a straight or branched chain alkyl group having 1 to 4, for example methyl, ethyl n-propyl, isopropyl, n-butyl, isobutyl, and tert-butyl. The term "$C_1$-$C_5$ alkyl" represented by $R_2$ means a straight or branched chain alkyl group having 1 to 5 preferably 1 to 3 carbon atoms, for example methyl, ethyl, n-propyl, isopropyl, n-butyl, isobutyl, n-pentyl and isopentyl. The term "$C_1$-$C_{10}$ alkylene" represented by A means a straight or branched

alkylene group having 1 to 10 carbon atoms, for example methylene, methylmethylene, dimethylmethylene, ethylene, 1-methylethylene, 2-methylethylene, 1-ethylethylene, 2-ethylethylene, 1,2-dimethylethylene, 1,1-dimethyl ethylene, 2,2-dimethylethylene, trimethylene, 1-methyltrimethylene, 2-methyltrimethylene, 3-methyltrimethylene, 2-ethyltrimethylene, 1,2-dimethyltrimethylene, 2,3-dimethyltrimethylene, tetramethylene, 1-methyltetramethylene, 2-methyltetramethylene, 3-methyltetramethylene, 4-methyltetramethylene, 1,1-dimethyltetramethylene, 2,2-dimethyltetramethylene, 3,3-dimethyltetramethylene, 4,4-dimethyltetramethylene, 1,2-dimethyltetramethylene, 1,3-dimethyltetramethylene, 1,4-dimethyltetramethylene, 2,3-dimethyltetramethylene, 2,4-dimethyltetramethylene, 3,4-dimethyltetramethylene, pentamethylene, 1-methylpentamethylene, 2-methylpentamethylene, 3-methylpentamethylene, 4-methylpentamethylene, 5-methylpentamethylene, 1,1-dimethylpentamethylene, 2,2-dimethylpentamethylene, 3,3-dimethylpentamethylene, 1,2-dimethylpentamethylene, 2,3-dimethylpentamethylene, hexamethylene, 1-methylhexamethylene, 2-methylhexamethylene, 3-methylhexamethylene, 4-methylhexamethylene, 5-methylhexamethylene, 6-methylhexamethylene, 1,1-dimethylhexamethylene, 2,2-dimethylhexamethylene, 3,3-dimethylhexamethylene, 1,2-dimethylhexamethylene, 1,3-dimethylhexamethylene, 1,4-dimethylhexamethylene,

2,3-dimethylhexamethylene, 1-ethylhexamethylene, 2-ethylhexamethylene, 3-ethylhexamethylene, heptamethylene, 1-methylheptamethylene, 2-methylheptamethylene, 3-methylheptamethylene, 4-methylheptamethylene, 1-ethylheptamethylene, 2-ethylheptamethylene, 3-ethylheptamethylene, 1,1-dimethylheptamethylene, 2,2-dimethylheptamethylene, 3,3-dimethylheptamethylene, 1,2-dimethylheptamethylene, 1,3-dimethylheptamethylene, 2,3-dimethylheptamethylene, octamethylene, 1-methyloctamethylene, 2-methyloctamethylene, 3-methyloctamethylene, 1-ethyloctamethylene, 2-ethyloctamethylene, 1,2-dimethyloctamethylene, 1,3-dimethyloctamethylene, 2,3-dimethyloctamethylene, nonamethylene, 1-methylnonamethylene, 2-methylnonamethylene, 3-methylnonamethylene, decamethylene.

The term "$C_1$-$C_5$ alkylene" represented by D means a straight or branched chain alkylene group having 1 to 5 carbon atoms, for example methylene, methylmethylene, dimethylmethylene, ethylmethylene, propylmethylene, butylmethylene, methylethylmethylene, methylpropylmethylene, ethylene, 1-methylethylene, 2-methylethylene, 1-ethylethylene, 2-ethylethylene, 1,2-dimethylethylene, 1,1-dimethylethylene, 2,2-dimethylethylene, trimethylene, 1-methyltrimethylene, 2-methyltrimethylene, 3-methyltrimethylene, tetramethylene, pentamethylene.

The term "cycloalkylene" represented by B and D contains from 3 to 10, preferably 5 or 6, ring carbon atoms, for example cyclopropylene, cyclobutylene, cyclopentylene, cyclohexylene etc. The hydroxy groups represented by $Y_1$, $Y_2$ and $Y_3$ may be, each independently converted by, for example, silylation, etherification or esterification to a protected hydroxy group.

Examples of suitable hydroxy-protecting groups may include: (1) trisubstituted silyl, for example trimethylsilyl, triethylsilyl, tri-n-butylsilyl, tert-butyldimethylsilyl, tribenzylsilyl, or triphenylsilyl, (2) 5- or 6- membered heterocyclic groups having an oxygen or sulphur atom in the ring and which may be unsubstituted or have one or more alkoxy substituents, for example 2-tetrahydrofuranyl, 2-tetrahydropyranyl, or 4-methoxytetrahydropyran-4-yl, (3) a lower alkyl group having one or more alkoxy or aralkoxy substituents, for example methoxy-2-propyl, 2-methoxyethoxymethyl, or benzyloxymethyl, (4) trityl group, (5) a lower alkanoyl group having 1 to 5 carbon atoms, for example acetyl, propionyl, butyryl or pivaloyl, (6) and aroyl group, for example benzoyl, 1-naphthoyl or 2-naphthoyl group, each of which may be unsubsituted or substituted by halogen atoms, a phenyl group, nitro group, or alkoxy group. Preferable hydroxy-protecting groups are lower alkanoyl groups.

The compound of the formula [I] in which $R_1$ is

a hydrogen atom may be converted to a pharmacologically acceptable salt form, if required. Acceptable salt forms may be: (1) salts of alkali or alkaline earth metals, for example sodium, potassium, magnesium or calcium, (2) ammonium salts, (3) quarternay ammonium salts, for example tetramethylammonium salts, tetraethylammonium salts or benzyltrimethylammonium salts, (4) salts of lower aliphatic amines, lower alicyclic amines or lower aralkylamines, for example methylamine, ethylamine, dimethylamine, diethylamine, trimethylamine, triethylamine, dicyclohexylamine, benzylamine, or α-phenylethylamine, (5) salts of heterocyclic amines, for example piperidine, morpholine, or pyridine, (6) salts of other amines, for example monoethanolamine, ethyl diethanolamine, or tris (hydroxymethyl) methylamine.

A tremendous amount of research in the synthetic organic chemistry, pharmacology and clinical medicine of prostaglandins has been performed since the discovery of prostaglandins.

In 1976, J. R. Vane et al. reported the isolation and biological effect of prostacyclin (prostaglandin $I_2$) [S. Moncada, R. Gryglewski, S Bunting, and J. R. Vane, Nature (London), 263, 633 (1976)].

Prostaglandin $I_2$ [III], which is shown below, has several excellent pharmacological activities, for example, hypotensive, vasodilating, antiallergic,

antiulcerogenic, antisecretory, and antithrombotic activities, and is expected to be useful in treating and preventing hypertention, asthma, ulcer,

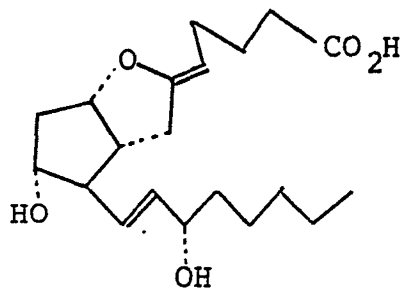

[III]

peripheral arterial diseases, cerebral ischemic attacks, myocardial infarcts and strokes, atherosclerosis arteriosclerosis and post-operative thrombosis.

However, the short half-life and chemical instability of prostaglandin $I_2$ is unsuitable for pharmaceutical use.

We have now discovered a series of novel bicyclooctane compounds [I] of this invention and their pharmaceutically acceptable salts, which have potent platelet anti-aggregation activity and hypotensive activity and are useful generally in treating and preventing thromboses and in treating hypertension.

In addition, the novel bicyclooctane compounds [I] have potent gastric antisecretory and antiulcerogenic activity and are useful in treating and preventing gastrointestinal ulcer. Furthermore, the novel bicyclooctane compounds [I] have a greater advantage in their chemical stability and easiness to handle, and

hence can be administered even orally.

The novel bicyclooctane compounds [I] and their pharmaceutically acceptable salts can effectively be administered orally in tablets, capsules, drops or syrups, or parenterally such as intravenously, subcutaneously, intramuscularly in sterile or suspensions.

The exact doses are depending on the age, weight, condition of the patient, the frequency and route of administration. For the puprose of treatment or prevention of thromboses, oral dosages are used in the range about 0.1 to 1000 mg, preferably about 0.1 to 100 mg per day.

For the purpose of treatment of ulcer, oral dosages are used in the range about 0.01~100 mg, preferably about 0.1 to 20 mg per day.

Accordingly, a basic object of the present invention is to provide novel and stable bicyclooctane compounds [I] having an antithrombotic activity, antiulcer activity.

Another object of this invention is to provide a process for producing those compounds [I]. These and other objects will be apparent to those skilled in the art to which the present invention pertains from the foregoing and subsequent descriptions.

The novel bicyclooctane compounds [I] of this invention can be prepared by the following methods:

Method (a): The novel bicyclooctane compounds of the formula [I]:

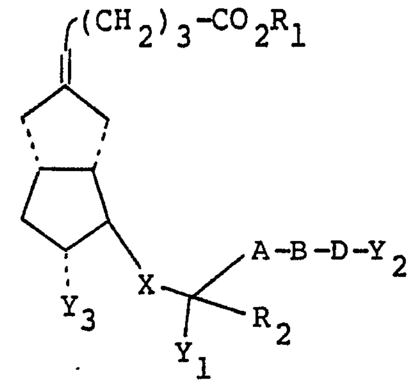

[I]

wherein $R_1$, $R_2$, A, B, D, X, $Y_1$, $Y_2$ and $Y_3$ are as defined above, can be preared by reacting a carbonyl compound of the formula [IV];

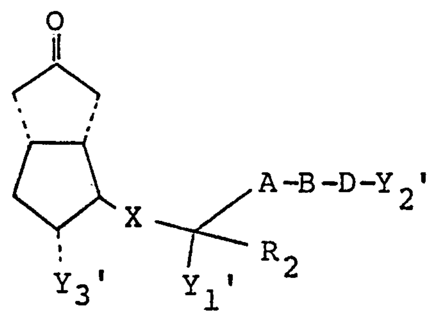

[IV]

wherein $R_2$, A, B, D and X are as defined above, and $Y_1'$, $Y_2'$ and $Y_3'$ are independently of each other a free or a protected hydroxy group, with a compound of the formula [V];

$$(Ph)_3P{=}CH{-}(CH_2)_3{-}CO_2M \qquad [V]$$

wherein M is a hydrogen atom or an alkali metal, for example, sodium, lithium or potassium, preferably sodium, and Ph is a phenyl group, and if necessary, subsequently one or more of the following reactions are carried out at any appropriate step in any desired

sequence: (1) separation of a mixture of isomers, (2) deprotection of a protected hydroxy group, (3) protection of a free hydroxy group, (4) esterification of carboxyl group, (5) hydrolysis of an esterified carboxy group.

Method (b): The novel bicyclooctane compounds of the formula [I], in which X represents trans vinylene group, may be also prepared by reacting an aldehyde compound of the formula [VI]:

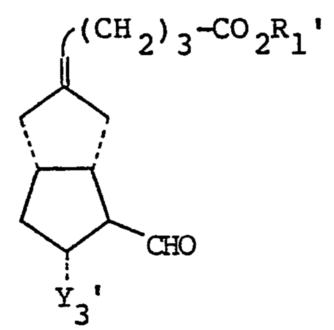

[VI]

wherein $Y_3'$ is as defined above, and $R_1'$ represents a $C_1$-$C_4$ alkyl group, with a compound of the formula [VII]:

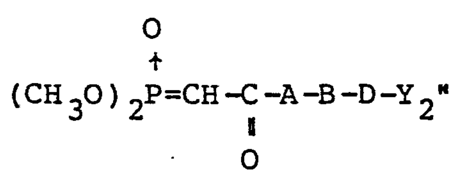

[VII]

wherein A, B and D are each as defined above, and $Y_2''$ is a free or a protected hydroxy group which may be easily replaced with a hydrogen by acidic conditions, and further reacting the carbonyl group of the resulting enone [VIII]:

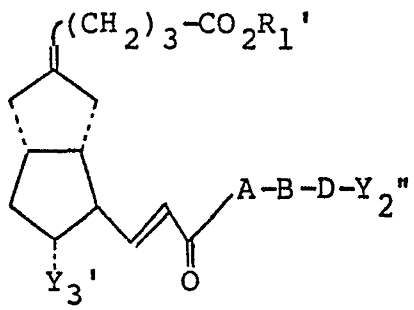

[VIII]

wherein $R_1'$, A, B, D, $Y_2''$ and $Y_3'$ are each as defined above, with a reducing agent or an organometallic compound of the formula [IX]:

$M'-R_2'$ [IX]

wherein $R_2'$ is a $C_1$-$C_5$ alkyl group and M' is a lithium atom or -Mg halo (halo is a halogen atom), and if necessary, subsequently one or more of the following reactions are carried out at any appropriate step in any desired sequence: (1) separation of a mixture of isomers, (2) deprotection of a protected hydroxy group, (3) protection of a free hydroxy group, (4) hydrolysis of an esterified carboxy group.

In the significances of the formula [VII], examples of the hydroxy protecting group which may be removed by acidic conditions, include (1) trisubstituted silyl, for example trimethylsilyl, triethylsilyl, tri-n-butylsilyl, tert-butyldimethylsilyl, tribenzylsilyl, or triphenylsilyl, (2) 5- or 6-membered heterocyclic groups having an oxygen or sulphur atom in the ring, for example 2-tetrahydrofuranyl, 2-tetrahydropyranyl, or 2-tetrahydrothiopyranyl, (3) a lower alkyl group having

one or more alkoxy or aralkoxy substituents, for example methoxymethyl, 1-ethoxyethyl, 2-methoxy-2-propyl, or 2-methoxyethoxymethyl (4) trityl group.

The Wittig reagents [V] and [VII] can be prepared according to the known method [E. J. Corey et al., J. Amer. Chem. Soc., 91 5675 (1969)].

The Wittig reaction can be carried out in a known manner in the presence of a solvent using 1-10 equivalents (preferably 2-6 equivalents) of the Wittig reagent [V] and [VII]. Examples of the solvent are ethers (e.g. diethyl ether, tetrahydrofuran, dioxane, dimethoxyethane), hydrocarbons (e.g. benzene, toluene, hexane), N,N dimethylformamide and dimethylsulfoxide. The reaction can be controlled with warming or cooling, usually at a temperature from 0°C to the boiling point of solvent, preferably at room temperature, depending upon the extent of the progress. The reaction time may vary depending upon the reaction temperature and the reagent to be used therein but it generally takes 1-24 hrs.

The carbonyl compound [VIII] can be converted into the corresponding alcohol compound by reacting the former with a reducing agent or an organometallic compound [IX].

For reduction, there may be used any known reducing agent which can reduce only ketonic carbonyl group without affectig ester group in an inert solvent

(e.g. diethyl ether, tetrahydrofuran, dimethoxyethane, pentane, hexane, benzene, toluene, methanol, ethanol) at a temperature ranging from -70°C to room temperature.

Examples of such reducing agents are metal borohydrides, for example sodium borohydride, potassium borohydride, zinc borohydride, lithium triisobutylborohydride and sodium trimethoxyborohydride, metal aluminum hydrides, for example lithium (tri-tert-butoxy)aluminum hydride, lithium ethoxy 1,1'-binaphthyl-2,2'-dioxyaluminum hydride, and aluminum alkoxides such as aluminum isopropoxide and diisobutylaluminum 2,6-di-tert-butyl-4-methylphenoxide.

The reaction conditions may vary depending upon the reaction temperature and the reducing agent to be used therein, but the reaction may be preferably carried out at a temperature from -70°C to 0°C, using 1 to 3 equivalents of reducing agents.

For alkylation, the reaction may be carried out with 1 to 5 equivalents of organometallic compound of the formula [IX], at a temperature from -70°C to room temperature in an inert solvent (e.g. ethers for example diethylether, tetrahydrofuran, and dimethoxyethane or hydrocarbons for example benzene, toluene, and hexane).

The organometallic compound [IX] can be usually prepared from the corresponding halide by the conventional procedures.

The bicyclooctane compound thus obtained can

be separated from the reaction mixture and purified by the conventional procedures, for example column or thin layer chromatography and optionally followed by esterification of a carboxy group, hydrolysis of an esterified carboxy group, protection of a free hydroxy group, or deprotection of protected hydroxy group.

The esterification of carboxy group, hydrolysis of ester group, protection of a free hydroxy group or deprotection of protected hydroxy group can be carried out by the conventional procedures [Protective Group in Organic Chemistry, edited by J. F. W. McOmie (1973), 95-143 and 183-215].

This invention further provides a pharmaceutical composition comprising at least one of the compounds of the formula [I] as an active ingredient and at least one pharmaceutically acceptable carrier or diluent.

For the preparation of pharmaceutical compositions, the compounds of the formula [I] may be mixed with conventional excipients, such as carriers, diluents, lubricants, fillers, binders, stabilizers or emulsifying agent (e.g. lactose, sucrose, calcium phosphate, starch, talcum, casein, magnesium stearate, methyl cellulose, polyglycols, tragacanth and the like).

The resulting mixture may be processed in a usual manner to tablets, capsules, pills, ampoules and the like.

The starting materials of this invention can be prepared by the reactions and procedures described and exemplified hereinafter.

The bicyclooctane compounds of the formula [IV], in which X represents an ethylene group or a vinylene group, are prepared by the sequence of transformations shown in Chart A.

[Chart A]

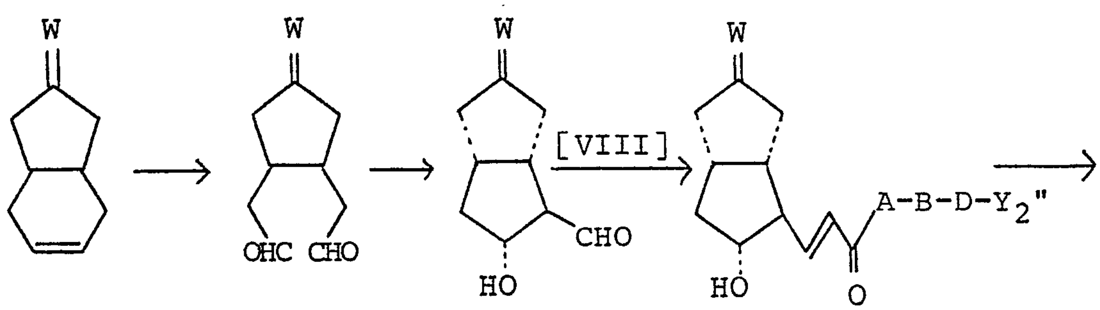


[X] [XI] [XII] [XIII]

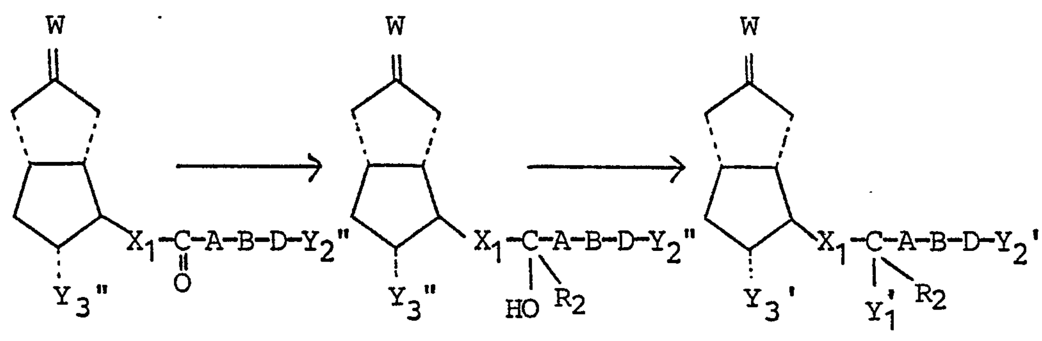

[XIV] [XV] [XVI]

wherein A, B, D, $R_2$, $Y_1'$, $Y_2'$, $Y_3'$ and $Y_2''$ are each as defined above and W is a protected carbonyl group, and $X_1$ is an ethylene group or a vinylene group and $Y_3''$ is a

free or protected hydroxy group which may be easily replaced with a hydrogen atom by acidic conditions.

The aldol compound [XII] is effectively obtained from the cyclohexene compound [X] by the oxidative cleavage to the dialdehyde compound [XI] followed by the intermolecular aldol reaction. [Japanese Patent Application Kokai (Laid-Open) No. 105,638/80].

The aldol compound [XII] thus obtained is converted by treatment with the Wittig reagent [VIII] to the enone compound [XIII] and if desired, followed by reduction of the double bond by conventional catalytic hydrogenation in the presence of palladium-on-carbon or similar catalyst and/or, if necessary, protection of the hydroxy group to the compound of the formula [XIV].

Reduction of the carbonyl group of the compound [XIV] with a metal hydride reducing agent or the reaction of the compound [XIV] with an organometallic compound [IX] affords the compound [XV] and deprotection of the carbonyl protecting group of the resulting compound [XV] and, if necessary, protection of the hydroxy group before and/or after deprotection of carbonyl protecting group gives the compound of the formula [XVI].

The bicyclooctane compounds of the formula [IV], in which X represents an ethynylene group, are prepared by the sequence of transformations shown in Chart B.

[Chart B]

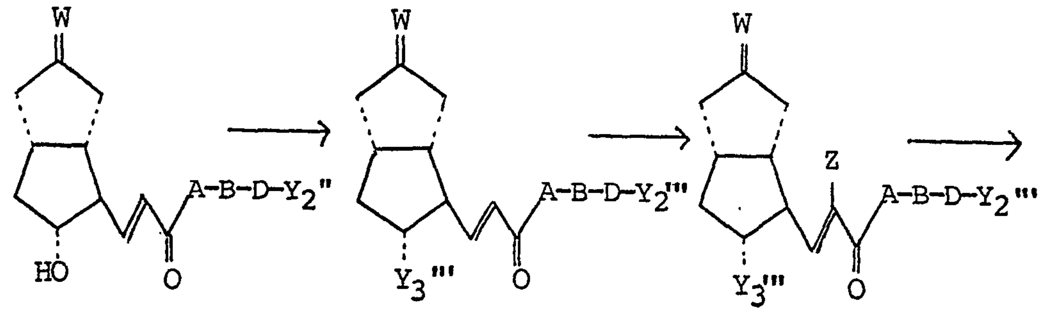

[XIII] [XVII] [XVIII]

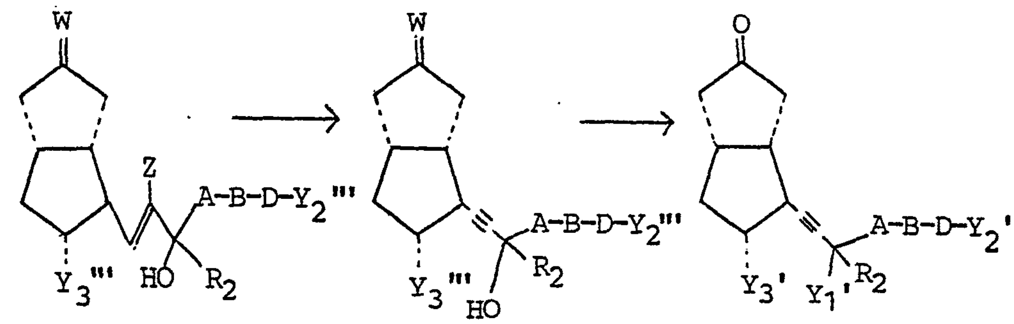

[XIX] [XX] [XXI]

wherein A, B, D, $R_2$, $Y_1'$, $Y_2'$, $Y_3'$, $Y_2''$ and W are each as defined above and $Y_2'''$ and $Y_3'''$ are the protected hydroxy group and Z is a halogen atom.

The compound of formula [XIII] obtained above (in Chart A) are converted to the compound of the formula [XVII] and then halogenated with bromine, chlorine or N-chlorosuccinimide to the dihalide. Dehydrohalogenation of the dihalide with an organic base such as pyridine yields the compound of the formula [XVIII]. Alternatively, the reaction of the compound of the formula [XVII] with pyridinium perbromide in the presence of an organic base such as pyridine may also

afford the compound of the formula [XVIII].

The reaction of the compound of the formula [XVIII] with a reducing agent or an organometallic compound [IX] gives the compound of the formula [XIX], and then dehydrohalogenation of the compound [XIX] with a strong organic base, for example potassium tert-butoxide or sodium methoxide may yield the compound of the formula [XX]. Deprotection of the carbonyl protecting group of the compound [XX] and, if necessary, protection of the hydroxy group before and/or after deprotection of the carbonyl protecting group gives the compound of the formula [XXI].

The bicyclooctane compound of the formula [VI] are prepared by the sequence of transformations shown in Chart C. [Japanese Patent Application Kokai (Laid-Open) No. 120,555/80]

[Chart C]

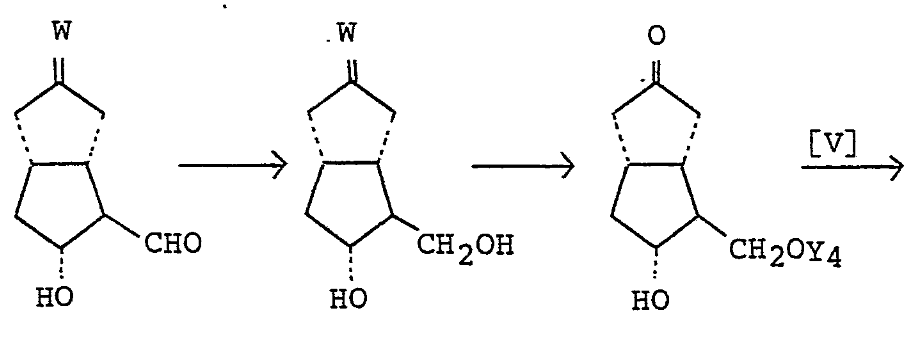


[XII] [XXII] [XXIII]

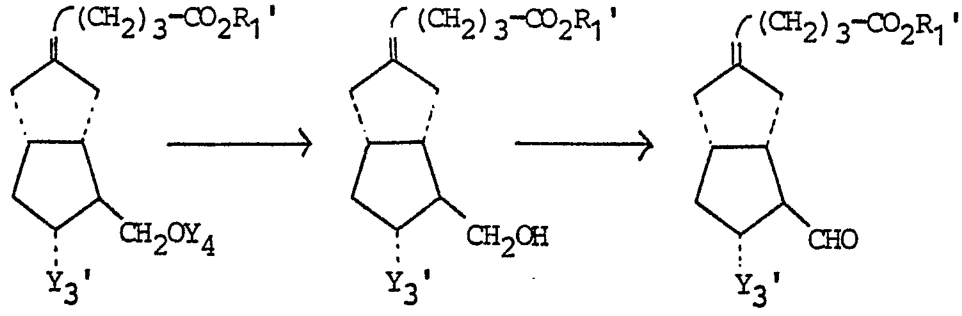

[XXIV] [XXV] [VI]

wherein $R_1'$, W and $Y_3'$ are each as defined above, $Y_4$ is a hydrogen atom or a hydroxy-protecting group such as trityl, pivaloyl or adamantoyl, which protects selectively a primary hydroxy group.

The compound [XXII] is obtained from the aldol [XII] by metal hydride reduction, for example sodium borohydride in methanol. The compound [XXIII] is obtained by deprotection of the carbonyl-protecting group of the compound [XXII], of which primay hydroxy group is selectively protected by, for example trityl chloride in pyridine and optionally followed by protection of the remaining secondary hydroxy group.

The compound [XXIII] is converted to the compound of the formula [XXIV], by the treatment with the Wittig reagent [V], followed by esterification and/or protection of the secondary hydroxy group. The aldehyde compound [VI] is obtained by selective deprotection of the primary hydroxy-protecting group of the compound [XXIV], followed by oxidation of the resulting alcoholic

compound [XXV] with an oxidizing agent, for example Collins reagent, pyridinium chlorochromate.

Examples of suitable hydroxy-protecting groups represented by $Y_1'$, $Y_2'$, $Y_3'$, $Y_2'''$ and $Y_3'''$ are the same as those represented by $Y_1$, $Y_2$ and $Y_3$.

The bicyclooctane compound [I] can exist in the form of various stereoisomers depending upon the conformation of the hydroxy group in the side chain attached to the bicyclooctane ring (at the 3'-position) and the double bond attached to the bicyclooctane ring (at the 7-position). For example, as to the typical compound [XXVI] of the formula [I], there are three isomers such as [XXVII], [XXVII] and [XXIX].

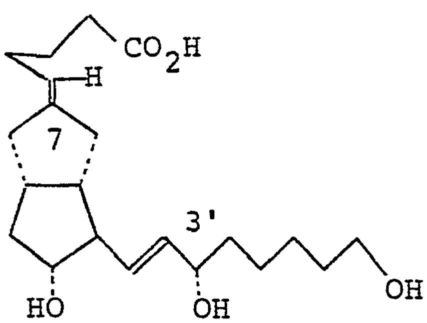

[XXVI]
(7E, 3'α)

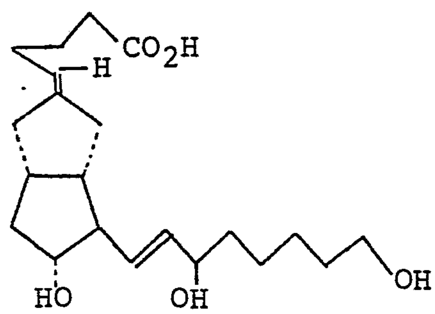

[XXVII]
(7E, 3'β)

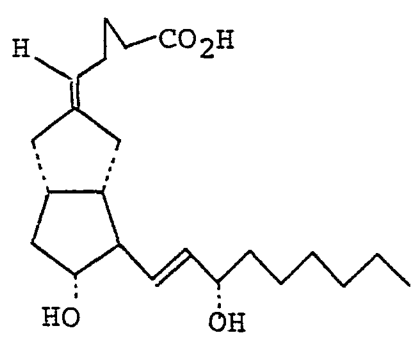

[XXVIII]
(7Z, 3'α)

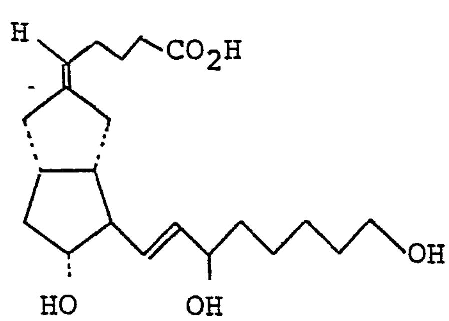

[XXIX]
(7Z, 3'β)

According to the present invention, the bicyclooctane compound [I] can be obtained as a mixture of stereoisomers, which can be easily separated by the conventional procedure (for example, column chromatography, thin layer chromatograph, etc) with high purity.

Although all of these stereoisomers are represented by a single formula [I], the present invention covers both the individual isomers and mixtures thereof.

Specific examples of the bicyclo compound [I] are the (7E, 3'α) isomer, the (7E, 3'β) isomer, the (7Z, 3'α) isomer, the (7Z, 3'β) isomer and a mixture of isomers of the following compounds.

2β-(3',8'-dihydroxy-trans-1'-octenyl)-3α-hydroxy-7-(4"-carboxybutylidene)-cis-bicyclo [3,3,0] octane.

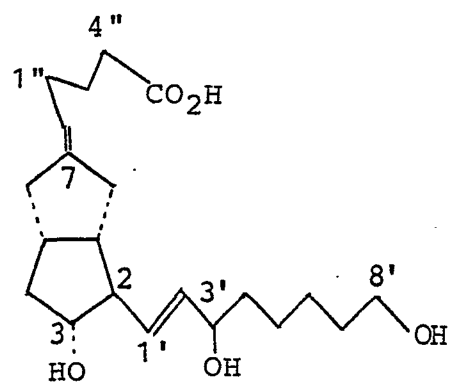

2β-(3',5'-dihydroxy-trans-1'-nonenyl)-3α-hydroxy-7-(4"-carboxybutylidene)-cis-bicyclo [3,3,0] octane.

2β-(3',6'-dihydroxy-6'-methyl-trans-1'-octenyl)-3α-hydroxy-7-(4"-carboxybutylidene)-cis-bicyclo [3,3,0] octane.

2β-(3',6'-dihydroxy-trans-1'-nonenyl)-3α-hydroxy-7-(4"-carboxybutylidene)-cis-bicyclo [3,3,0] octane.

2β-(3',7'-dihydroxy-trans-1'-nonenyl)-3α-hydroxy-7-(4"-carboxybutylidene)-cis-bicyclo [3,3,0] octane.

2β-(3',7'-dihydroxy-7'-methyl-trans-1'-nonenyl)-3α-hydroxy-7-(4"-carboxybutylidene)-cis-bicyclo [3,3,0] octane.

2β-(3',8'-dihydroxy-1'-octyl)-3α-hydroxy-7-(4"-carboxybutylidene)-cis-bicyclo [3,3,0] octane.

2β-(3',8'-dihydroxy-1'-octynyl)-3α-hydroxy-7-(4"-carboxybutylidene)-cis-bicyclo [3,3,0] octane.

2β-(3',8'-dihydroxy-3'-methyl-trans-1'-octenyl)-3α-hydroxy-7-(4"-carboxybutylidene)-cis-bicyclo [3,3,0] octane.

2β-(3',8'-dihydroxy-5'-methyl-trans-1'-octenyl)-3α-hydroxy-7-(4"-carboxybutylidene)-cis-bicyclo [3,3,0] octane.

2β-(3',8'-dihydroxy-trans-1'-nonenyl)-3α-hydroxy-7-(4"-carboxybutylidene)-cis-bicyclo [3,3,0] octane.

2β-(3',8'-dihydroxy-1'-nonynyl)-3α-hydroxy-7-(4"-carboxybutylidene)-cis-bicyclo [3,3,0] octane.

2β-(3',9'-dihydroxy-trans-1'-nonenyl)-3α-hydroxy-7-(4"-carboxybutylidene)-cis-bicyclo [3,3,0] octane.

2β-(3',9'-dihydroxy-4',4'-dimethyl-trans-1'-nonenyl)-3α-hydroxy-7-(4"-carboxybutylidene)cis-bicyclo [3,3,0] octane.

2β-(3',9'-dihydroxy-4',5'-dimethyl-trans-1'-nonenyl)-3α-hydroxy-7-(4"-carboxybutylidene)-cis-bicyclo [3,3,0] octane.

2β-(3',11'-dihydroxy-trans-1'-dodecenyl)-3α-hydroxy-7-(4"-carboxybutylidene)-cis-bicyclo [3,3,0] octane.

2β-(3',8'-dihydroxy-4'-methyl-6'-yn-trans-1'-octenyl)-3α-hydroxy-7-(4"-carboxybutylidene)-cis-bicyclo [3,3,0] octane.

2β-(3',8'-dihydroxy-4'-methyl-cis-6'-trans-1'-octadienyl)-3α-hydroxy-7-(4"-carboxybutylidene)-cis-bicyclo [3,3,0] octane.

2β-[3'-hydroxy-4'-(4"-hydroxybutoxy)-trans-1'-butenyl)-3α-hydroxy-7-(4"-carboxybutylidene)-cis-bicyclo [3,3,0] octane.

2β-[3'-hydroxy-4'-(m-hydroxymethyl-phenyl)-trans-1'-butenyl]-3α-hydroxy-7-(4"-carboxybutylidene)-cis-bicyclo [3,3,0] octane.

2β-[3'-hydroxy-4'-(m-hydroxymethyl-phenoxy)-trans-1'-butenyl]-3α-hydroxy-7-(4"-carboxybutylidene)-cis-bicyclo [3,3,0] octane.

2β-[3'-hydroxy-4'-(4"-hydroxy-cyclohexyl)-trans-1'-butenyl]-3α-hydroxy-7-(4"-carboxybutylidene)-cis-bicyclo [3,3,0] octane.

2β-(3',8'-diacetoxy-trans-1'-octenyl)-3α-acetoxy-7-(4"-methoxycarbonylbutylidene)-cis-bicyclo [3,3,0] octane.

2β-(3',8'-diacetoxy-octyl)-3α-acetoxy-7-(4"-methoxycarbonylbutylidene)-cis-bicyclo [3,3,0] octane.

2β-(3',10'-dihydroxy-trans-1'-decenyl)-3α-hydroxy-7-(4"-carboxybutylidene)-cis-bicyclo [3,3,0] octane.

The following Examples are given for the purpose of illustration of the preparation of starting materials embodying the invention in accordance with the above Charts (Referential Examples 1 to 17) and of the preparation of compounds embodying the invention (Examples 1 to 12), and they are not intended to limit the scope of the invention thereto.

Referential Example 1

To a tetrahydrofuran (THF, 500 ml) solution of dimethyl methylphosphonate (50 g) was added a 1.6 M hexane solution of n-butyl lithium (300 ml) at -60° under nitrogen. To this mixture was added a THF solution (100 ml) of ε-caprolactone (27 g) and stirred at the same temperature for 2 hrs and 0°C for 2 hrs. The mixture was condensed in vacuo after addition of acetic acid (26 g). The residue was dissolved with water and

extracted with chloroform. The extract was dried and concentrated in vacuo. After the residue was column chromatographed on silica gel using ethyl acetate as an eluent, there was obtained dimethyl 2-oxo-7-hydroxyheptyl phosphonate (40 g) as an oil.

NMR $\delta(CDCl_3)$: 3,75(6H, d), 3.08(2H, d), 2.60(2H, t)

Reference Example 2

In the same manner as Referential Example 1, the following compounds were obtained:

dimethyl 2-oxo-7-trimethylsilyloxy-octyl-phosphonate

NMR $\delta(CDCl_3)$: 3 .76(6H, d), 3.06(2H, d), 2.60(2H, t), 1.10(3H, d), 0.1(9H, s)

dimethyl 2-oxo-10-trimethylsilyloxy-undecyl phosphonate

NMR $\delta(CDCl_3)$: 4.10(1H, m) 3.78(6H, d) 3.07(2H, d) 2.60(2H, t) 0.09(9H, s)

dimethyl 2-oxo-3,3-dimethyl-8-hydroxy-octyl phosphonate

NMR $\delta(CDCl_3)$: 3.79(6H, d), 3.12(2H, d), 1.17(6H)

dimethyl 2-oxo-3,4-dimethyl-8-hydroxy-octyl phosphonate

NMR $\delta(CDCl_3)$: 3.79(6H, d), 3.12(2H, d), 1.17(3H, d) 1.14(3H, d)

dimethyl 2-oxo-8-tetrahydropyranyloxy-octyl phosphonate

NMR $\delta(CDCl_3)$: 4.6-4.4(1H, m), 3,72(6H, d), 3.0(2H, d) 2.57(2H, t)

dimethyl 2-oxo-9-tetrahydropyranyloxy-nonyl phosphonate

NMR $\delta(CDCl_3)$: 4.6-4.4(1H, m), 3.76(6H, d) 3.09(2H, d), 2.60(2H, t)

dimethyl 2-oxo-3-(4'-tetrahydropyranyloxy-butoxy)-propyl-phosphonate

NMR $\delta(CDCl_3)$: 4.75-4.6(1H, m) 3.80(6H, d) 3.03(2H, d) 2.47(2H, d)

dimethyl 2-oxo-3-(4'-tetrahydropyranyloxy-cyclohexyl)-propyl phosphonate

NMR $\delta(CDCl_3)$: 4.67-4.47(1H, m), 4.07(2H, s) 3.75(6H, d), 3.15(2H, d)

dimethyl 2-oxo-4-methyl-7-tetrahydropyranyloxy-heptyl phosphonate

NMR $\delta(CCl_4)$: 4.6-4.45(1H, m) 3.73(6H, d) 3.07(2H, d) 2.52(2H, t) 0.9(3H, d)

Referential Example 3

A solution of 8,8-ethylenedioxy-cis-bicyclo-[4,3,0]non-3-ene (1.0 g) in methanol (50 ml) was cooled to -50°C and subjected to a stream of ozonized oxygen. After the starting material was disappeared, 35 ml of dimethylsulfide was added and the mixture was stirred for 2 hrs at -15°C to -5°C.

The mixture was then concentrated by introduction of a stream of nitrogen to give oily cis-1,2-diformylmethyl-4,4-ethylenedioxy-cyclopentane

IR $\nu_{cm^{-1}}^{film}$: 1725

NMR $\delta(CDCl_3)$: 3.8, 9.7

The dialdehyde (1.0 g) obtained above was dissolved in methanol (30 ml) and cooled at -15°C to -5°C.

An aqueous solution of potassium hydroxide (5%, 10 ml) was added, and the mixture was stirred for 13 min, at the same temperature.

To this mixture was added a saturated solution of sodium chloride and ethyl acetate. The organic layer was separated, washd with water, dried and concentrated under reduced pressure at a low temperature to give oily 2β-formyl-3α-hydroxyl-7,7-ethylenedioxy-cis-bicyclo-[3,3,0]octane as a main product.

IR $\nu_{cm^{-1}}^{film}$: 3400, 1710

NMR $\delta(CDCl_3)$: 3.85, 9.75

Referential Example 4

A solution of 2β-formyl-3α-hydroxy-7,7-ethylenedioxy-cis-bicyclo[3,3,0]octane (15 g) in dry tetrahydrofuran (30 ml) was added at 3°C under nitrogen to a tetrahydrofuran solution (1 l) of the ylide prepared with dimethyl 2-oxo-7-hydroxy-heptyl phosphonate

(20 g) and sodium hydride (60%, 2.64 g). The mixture was stirred for 2 hr at room temperature and poured into water and then extracted with ethyl acaetate. The extract was washed with aqueous sodium chloride solution, dried and concentrated in vacuo. The residue was chromatographed on silica gel to give 2β-(8'-hydroxy-3'-oxo-trans-1'-octenyl)-3α-hydroxy-7,7-ethylenedioxy-cis-bicyclo[3,3,0]octane (4.8 g).

IR $\nu_{cm^{-1}}^{film}$: 3400, 1690, 1685, 1625

NMR $\delta(CDCl_3)$: 6.75 (1H,d, d), 6.12(1H, d), 3,87(4H, s)

Referential Example 5

In the same manner as Referential Example 4, the following compounds were obtained:

2β-(8'-hydroxy-3'-oxo-trans-1'-nonenyl)-3α-hydroxy-7,7-ethylenedioxy-cis-bicyclo[3,3,0]octane

IR $\nu_{cm^{-1}}^{film}$: 3450-3400, 1690(sh), 1665, 1625

NMR $\delta(CDCl_3)$: 6,76(1H, d, d) 6.13(1H, d) 3,90(4H, s) 1.16(3H, d)

2β-(11'-hydroxy-3'-oxo-trans-1'-dodecenyl)-3α-hydroxy-7,7-ethylenedioxy-cis-bicyclo[3,3,0]octane

NMR $\delta(CDCl_3)$: 6.80(1H, d, d) 6.20(1H, d) 1.18(3H, d)

2β-(9'-hydroxy-4',4'-dimethyl-3'-oxo-trans-1'-nonenyl)-3α-hydroxy-7,7-ethylenedioxy-cis-bicyclo[3,3,0]octane

NMR $\delta(CDCl_3)$: 6.80(1H, d, d) 6.28(1H, d)
3,90(4H, s) 1.11(6H)

2β-(9'-hydroxy-4',5'-dimethyl-3'-oxo-trans-1'-nonenyl)-3α-hydroxy-7,7-ethylenedioxy-cis-bicyclo[3,3,0]-octane

NMR $\delta(CDCl_3)$: 6.80(1H, d, d) 6.23(1H, d)
3,90(4H, s) 10(6H, d)

2β-(9'-tetrahydropyranyloxy-3'-oxo-trans-1'-nonenyl)-3α-hydroxy-7,7-ethylenedioxy-cis-bicyclo[3,3,0]octane

NMR $\delta(CDCl_3)$: 6.75(1H, d, d) 6.12(1H, d) 4.5(2H, m)
3,87(4H, s)

2β-(10'-tetrahydropyranyloxy-3'-oxo-trans-1'-decenyl)-3α-hydroxy-7,7-ethylenedioxy-cis-bicyclo-[3,3,0]octane

NMR $\delta(CDCl_3)$: 6.76(1H, d, d) 6.13(1H, d)
4.5(2H, m) 3.88(4H, s)

2β-(8'-tetrahydropyranyloxy-5'-methyl-3'-oxo-trans-1'-octenyl)-3α-hydroxy-7,7-ethylenedioxy-cis-bicyclo [3,3,0]octane

NMR $\delta(CDCl_3)$: 4.7-4.5(1H, br) 3.92(4H, s)
0.93(3H, d)

Referential Example 6

An ethyl acetate solution (20 ml) of 2β-[8'-hydroxy-3'-oxo-trans-1'-octenyl]-3α-hydroxy-7,7-ethylenedioxy-cis-bicyclo[3,3,0]octane (1.0 g) was hydrogenated with 5% Pd-C (0.3 g) under atmospheric pressure. The mixture was filtered to remove the catalyst, and the filtrate was concentrated to give oily 2β-[8'-hydroxy-3'-oxo-octyl]-3α-hydroxy-7,7-ethylenedioxy-cis-bicyclo[3,3,0]octane (0.9 g).

IR $\nu_{cm^{-1}}^{film}$: 3400, 1705

NMR $\delta(CDCl_3)$: 3.9(4H, s) 3.6(2H, t)

Referential Example 7

A mixture of 2β-(8'-hydroxy-3'-oxo-trans-1'-octenyl)-3α-hydroxy-7,7-ethylenedioxy-cis-bicyclo[3,3,0]-octane (0.8 g), dichloromethane (10 ml), 2,3 dihydropyran (0.8 g) and pyridinium p-toluenesulfonic acid was stirred for 17 hr at room temperature and then poured into saturated sodium bicarbonate solution and separated. The organic layer was dried and concentrated in vacuo to give oily 2β-(8'- tetrahydropyranyloxy-3'-oxo-trans-1'-octenyl-3α-tetrahydropyranyloxy-7,7-ethylenedioxy-cis-bicyclo[3,3,0]octane (1.6 g).

NMR $\delta(CDCl_3)$: 6.73 (1H, d, d), 6.13(1H, d)
5.0-4.5(2H, m), 3.88(4H, s)

According to the same manner described abovce, the following compounds were obtained.

2β-(8'-tetrahydropyranyloxy-3'-oxo-trans-1'-nonenyl)-3α-tetrahydropyranyloxy-7,7-ethylenedioxy-cis-bicyclo-[3,3,0]octane

NMR $\delta(CDCl_3)$: 6.76(1H, d, d), 6.13(1H, d)
4.7-4.5(2H, m), 3.87(4H, s)

2β-(11'-tetrahydropyranyloxy-3'-oxo-trans-1'-dodecenyl)-3α-tetrahydropyranyloxy-7,7-ethylenedioxy-cis-bicyclo-[3,3,0]octane

NMR δ($CDCl_3$): 6.80(1H, d, d) 6.20(1H, d)
4.70(2H, m) 1.18(3H, d)

Referntial Example 8

To a pyridine solution (15 ml) of 2β-(8'-tetrahydropyranyloxy-3'-oxo-trans-1'-nonenyl)-3α-tetrahydropyranyloxy-7,7-ethylenedioxy-cis-bicyclo[3,3,0]octane (1.57 g, Referential example 7) was added pyridinium perbromide (3 g) and the mixture was stirred for 17 hr. After addition of ethyl acetate, the mixture was filtrered. The filtrate was condensed under reduced pressure, and the residue was extracted with ethyl acetate. After the organic layer was dried and concentrated, these was obtained 2β-(8'-tetrahydropyranyloxy-3'-oxo-2'-bromo-trans-1'-nonenyl)-3α-tetrahydropyranyloxy-7,7-ethylenedioxy-cis-bicyclo[3,3,0]octane (2g).

NMR δ($CDCl_3$): 7.03(1H, d, d), 4.8-4.5(1H, m)
3.90(4H, s), 1.12(3H, d)

Referential Example 9

Into a THF solution (20 ml) of 2β-(8'-hydroxy-3'-oxo-trans-1'-octenyl)-3α-hydroxy-7,7-ethylenedioxy-cis-bicyclo[3,3,0]octane (1.0 g, Referential example 4) was added a solution of methylmagnesium iodide prepared from

magnesium ribbon (0.48 g) and methyliodide (2.8 g) in dry ethyl ether (25 ml). After being stirred at 5° for 0.5 hr and then at room temperature for 1 hr, the mixture was poured into saturated ammonium chloride solution and extracted with ethyl acetate. The organic layer was washed with brine, dried and concentrated in vacuo.

The residue was chromatographed on silica gel to give an oily 2β-(3',8'-dihydroxy-3'-methyl-trans-1'-octenyl)-3α-hydroxy-7,7-ethylenedioxy-cis-bicyclo[3,3,0]-octane (0.78 g).

IR $\nu_{cm^{-1}}^{film}$: 3400-3350.

NMR $\delta(CDCl_3)$: 5.7-5.4(2H, m), 3.90(4H, s)
3.60(2H, m), 1.25(3H, s)

Referential Example 10

A methanolic solution (60 ml) of 2β-[8'-tetrahydropyranyloxy-3'-oxo-trans-1'-octenyl]-3α-tetrahydropyranyloxy-7,7-ethylenedioxy-cis-bicyclo[3,3,0]octane (0.8 g, Referential example 7) was treated with sodium borohydride (0.3 g) with ice cooling.

Stirring was continued for 2 hrs and the excess reducing agent was quenched with acetone, and then the mixture was concentrated under reduced pressure. To the residue was added aqueous ammonium chloride, and the mixture was extracated with ethyl acetate.

The organic layer was dried and evaporated to give oily 2β-[8'-tetrahydropyranyloxy-3'-hydroxy-trans-1'-octenyl]-3α-tetrahydropyranyloxy-7,7-ethylenedioxy-cis-bicyclo[3,3,0]octane (0.8 g).

NMR δ($CDCl_3$): 5.65-5.45(2H, m), 5.0-4.5(2H, m) 3,87(4H, s)

According to the same procedure described above, there was obtained 2β-(3',8'-dihydroxy-octyl)-3α-hydroxy-7,7-ethylenedioxy-cis-bicyclo[3,3,0]octane from the corresponding ketone (Referential example 6).

NMR δ($CDCl_3$): 3.88(4H, s), 3.9-3.45(4H, m)

Referential Example 11

A toluene solution (50 ml) of 2β-(8'-tetrahydropyranyloxy-3'-oxo-trans-1'-nonenyl)-3α-tetrahydropyranyloxy-7,7-ethylenedioxy-cis-bicyclo[3,3,0]octane (2.14 g Referential example 7) was treated with a THF slution (10 ml) of 1M lithium triisobutylborohydride at -50°C under nitrogen atmosphere. Stirring was continued for 1 hr and the excess reducing agent was quenched with acetone, and then the mixture was poured into water and extracted with ethyl acetate. The organic layer was dried and concentrated to afford 2β-(8'-tetrahydropyranyloxy-3'-hydroxy-trans-1'-nonenyl)-3α-tetrahydropyranyloxy-7,7-ethylenedioxy-cis-bicyclo[3,3,0]octane (2.0 g).

NMR δ($CDCl_3$): 5.63-5.37(2H, m), 4.76-4.5(2H, m) 3.87(4H, s) 1.10(3H, d)

In the same manner as above, there were

obtained the following compounds from the corresponding ketones (Referential example 5, 7 or 8).

2β-(11'-tetrahydropyranyloxy-3'-hydroxy-trans-1'-dodecenyl)-3α-tetrahydropyranyloxy-7,7-ethylenedioxy-cis-bicyclo[3,3,0]octane

2β-(3',9'-dihydroxy-4',4'-dimethyl-trans-1'-nonenyl)-3α-hydroxy-7,7-ethylenedioxy-cis-bicyclo[3,3,0]octane

2β-(3',9'-dihydroxy-4',5'-dimethyl-trans-1'-nonenyl)-3α-hydroxy-7,7-ethylenedioxy-cis-bicyclo[3,3,0]octane

2β-(9'-tetrahydropyranyloxy-3'-hydroxy-trans-1'-nonenyl)-3α-hydroxy-7,7-ethylenedioxy-cis-bicyclo[3,3,0]-octane

2β-(10'-tetrahydropyranyloxy-3'-hydroxy-trans-1'-decenyl)-3α-hydroxy-7,7-ethylenedioxy-cis-bicyclo[3,3,0]-octane

2β-(8'-tetrahydropyranyloxy-5'-methyl-3'-hydroxy-trans-1'-octenyl)-3α-hydroxy-7,7-ethylenedioxy-cis-bicyclo-[3,3,0]octane

2β-(8'-tetrahydropyranyloxy-3'-hydroxy-2'-bromo-trans-1'-nonenyl)-3α-tetrahydropyranyloxy-7,7-ethylenedioxy-cis-bicyclo[3,3,0]octane

Referential Example 12

A mixture of 2β-(8'-tetrahydropyranyloxy-3'-hydroxy-trans-1'-octenyl)-3α-tetrahydropyranyloxy-7,7-ethylenedioxy-cis-bicyclo[3,3,0]octane (0,8 g, Referential example 10), acetic anhydride (2 ml) and

pyridine (2 ml) was stirred for 17 hr at room temperature. After addition of water, the mixture was extracted with ethyl acetate. The extract was washed with diluted hydrochloric acid, saturated sodium bicarbonate and brine, and dried. After evaporation of the solvent, there was obtained 2β-(8'-tetrahydropyranyloxy-3'-acetoxy-trans-1'-octenyl)-3α-tetrahydropyranyloxy-7,7-ethylenedioxy-cis-bicyclo[3,3,0]octane (1.0g)

NMR δ($CDCl_3$): 5.9-5.4(2H, m), 5,35-5.1(1H, m)
3.85(4H, s), 2.0(3H, s)

In the same manner as above there was obtained the following compound.

2β-(8'-tetrahydropyranyloxy-3'-acetoxy-trans-1'-nonenyl)-3α-tetrahydropyranyloxy-7,7-ethylenedioxy-cis-bicyclo[3,3,0]octane

NMR δ($CDCl_3$): 5.7-5.4(2H, m), 5.4-5.1(1H, m)
4.8-4.5(2H, m), 3.87(4H, s),
2.0(3H, s), 1.1(3H, d)

Referential Example 13

To a tert-butanol solution (10 ml) of 2β-(8'-tetrahydropyranyloxy-3'-hydroxy-2'-bromo-trans-1'-nonenyl)-3α-tetrahydropyranyloxy-7,7-ethylenedioxy-cis-bicyclo-[3,3,0]octane (1.63 g, Referential example 11) was added potassium tert-butoxide (1.2 g) and the mixture was stirred for 17 hr at room temperature. After evaporation of the mixture in vacuo, water and diethyl ether

was added to the residue. After separation, the organic layer was dried, concentrated and chromatographed on silica gel to give an oily 2β-(8'-tetrahydropyranyloxy-3'-hydroxy-1'-nonenyl)-3α-tetrahydropyranyloxy-7,7-ethylenedioxy-cis-bicyclo[3,3,0]octane (0.45 g)

NMR δ($CDCl_3$): 4.73-4.5(2H, br), 4.35-4.12(1H, m)
3.85(4H, s), 1.18-1.09(3H, 2d)

Referential Example 14

2β-(8'-tetrahydropyranyloxy-3'-acetoxy-trans-1'-octenyl)-3α-tetrahydropyranyloxy-7,7-ethylenedioxy-cis-bicyclo[3,3,0]octane (1.0 g, Referential example 12) was dissolved in a mixed solvent of acetic acid (9 ml), water (3 ml) and tetrahydrofuran (3 ml). The mixture was stirred for 4 hr at 60°C and condensed under reduced pressure, after addition of water and ethyl acetate, the organic layer was separated and dried and concentrated. The residue was chromatographed on silica gel to give a less polar 2β-(8'-hydroxy-3'β-acetoxy-trans-1'-octenyl)-3α-hydroxy-cis-bicyclo[3,3,0]octan-7-one (0.29 g) and a more polar 2β-(8'-hydroxy-3'α-acetoxy-trans-1'-octenyl)-3α-hydroxy-cis-bicyclo[3,3,0]octan-7-one (0.35 g)

NMR δ($CDCl_3$) 3'β: 5.9-5.4(2H, m) 5.35-5.1(1H, m)
2.0(3H, s)
3'α: 5.95-5.43(2H, m) 5.33-5.0(1H, m)
2.0(3H, s)

In the same manner as above, there were

obtained the following compounds from the corresponding ketals (Referential examples 10, 11, 12 and 13).

2β-(8'-hydroxy-3'-acetoxy-trans-1'-nonenyl)-3α-hydroxy-cis-bicyclo[3,3,0]octan-7-one

NMR δ($CDCl_3$): 5.6-5.4(2H, m) 5.3-5.0(1H, m) 4.0-3.6(2H, m) 2.01(3H, s) 1.15(3H, d)

2β-(3'α,11'-dihydroxy-trans-1'-dodecenyl)-3α-hydroxy-cis-bicyclo[3,3,0]octan-7-one

NMR δ($CDCl_3$): 5.52-5.42(2H, m) 4.19-3.63(3H, m) 1.17(3H, d)

2β-(3'β,11'-dihydroxy-trans-1'-dodecenyl)-3α-hydroxy-cis-bicyclo[3,3,0]octan-7-one

NMR δ($CDCl_3$): 5.57-5.47(2H, m) 4.13-3.58(3H, m) 1.17(3H, d)

2β-(3'α,9'-dihydroxy-4',4'-dimethyl-trans-1'-nonenyl)-3α-hydroxy-cis-bicyclo[3,3,0]octan-7-one

NMR δ($CDCl_3$): 5.6-5.4(2H, m) 4.14-3.72(4H, m) 0.90(6H)

2β-(3'β,9'-dihydroxy-4',4'-dimethyl-trans-1'-nonenyl)-3α-hydroxy-cis-bicyclo[3,3,0]octan-7-one

NMR δ($CDCl_3$): 5.65-5.55(2H, m), 4,22-3,83(4H, m) 0.90(6H)

2β-(9',3'α-dihydroxy-4',5'-dimethyl-trans-1'-nonenyl)-3α-hydroxy-cis-bicyclo[3,3,0]octan-7-one

MNR δ($CDCl_3$): 5.7-5.5(2H, m) 4.23-3,80(4H, m) 0.90(3H, d) 0.88(3H, d)

2β-(9',3'β-dihydroxy-4',5'-dimethyl-trans-1'-nonenyl)-3α-hydroxy-cis-bicyclo[3,3,0]octan-7-one

NMR δ($CDCl_3$): 5.6-5.4(2H, m) 4.08-3.58(4H, m) 0.95(3H, d) 0.85(3H, d)

2β-(9',3'α-dihydroxy-trans-1'-nonenyl)-3α-hydroxy-cis-bicyclo[3,3,0]octan-7-one

NMR δ($CDCl_3$): 5.6-5.4(2H, m), 4.2-3.6(4H, m)

2β-(9',3'β-dihydroxy-trans-1'-nonenyl)-3α-hydroxy-cis-bicyclo[3,3,0]octan-7-one

NMR δ($CDCl_3$): 5,65-5.4(2H, m) 4.15-3.55(4H, m)

2β-(10',3'-dihydroxy-trans-1'-decenyl)-3α-hydroxy-cis-bicyclo[3,3,0]octan-7-one

NMR δ($CDCl_3$): 5.6-5.4(2H, m), 4.1-3.55(4H, m)

2β-(8',3'-dihydroxy-1'-nonenyl)-3α-hydroxy-cis-bicyclo[3,3,0]octan-7-one

NMR δ($CDCl_3$): 4.5-4.0(2H, m) 4.0-3.5(1H, m) 1.18(3H, d)

2β-(8',3'-dihydroxy-octyl)-3α-hydroxy-cis-bicyclo-[3,3,0]octan-7-one

NMR δ($CDCl_3$): 3.9-3.7(1H, m) 3.7-3.4(3H, m)

2β-(8',3'α-dihydroxy-5'-methyl-trans-1'-octenyl)-3α-hydroxy-cis-bicyclo[3,3,0]octan-7-one

NMR δ($CDCl_3$): 5.65-5.45(2H, m) 4.3-3.6(4H, m)

2β-(8',3'β-dihydroxy-5'-methyl-trans-1'-octenyl)-3α-hydroxy-cis-bicyclo[3,3,0]octan-7-one

NMR δ($CDCl_3$): 5.7-5.45(2H, m) 4.2-3.55(4H, m)

Referential Example 15

2β-(3',8'-dihydroxy-3'-methyl-trans-1'-octenyl)-3α-hydroxy-7,7'-ethylenedioxy-cis-bicyclo[3,3,0]octane (1.1 g, Referential example 9) was dissolved in a mixed solvent of acetic acid (6 ml), water (2 ml) and tetrahydrofuran (2 ml) and further a $KH_2PO_4$-$Na_2HPO_4$ buffer solution (10 ml, pH 7.0). The mixture was stirred for 2 hr at 50°C and then poured into saturated sodium bicarbonate. After extracting with ethyl acetate, the organic layer was dried and concentrated in vacuo. The residue was chromatogaraphed on silica gel to afford an oily 2β-(3',8'-dihydroxy-3'-methyl-trans-1'-octenyl)-3α-hydroxy-cis-bicyclo[3,3,0]-octan-7-one (0.37 g).

NMR δ($CDCl_3$): 5.7-5.45(2H, m) 4.0-3.75(1H, m)
3.70(2H, m) 1.27(3H, s)

Referential Example 16

An ethanolic solution (300 ml) of 2β-formyl-3α-hydroxy-7,7-ethylenedioxy-cis-bicyclo[3,3,0]octane (10 g, obtained in Referential Example 1) was treated with sodium borohydride (10 g) at -10°C to o°C under nitrogen. The mixture was stirred for 1 hr at the same temperature and aqueous ammonium chloride was added. After extraction with ethyl acetate, the extract was dried and concentrated in vacuo. The residue was dissolved in a mixed solvent of 10 ml of acetic acid and 10ml of water and heated at 80°C for 10 min. The mixture was concentrated in vacuo to give 2β-hydroxymethyl-

3α-hydroxy-cis-bicyclo[3,3,0]octan-7-one.

Trityl chloride (11.3 g) was added to a pyridine solution (60 ml) of 2β-hydroxymethyl-3α-hydroxy-cis-bicyclo[3,3,0]octan-7-one (5.75 g). The mixture was stirred for 2.5 hrs at reflux and then poured into water and extracted with ethyl acetate. The extract was washed with water, dried and concentrated to give oily 2β-trityloxymethyl-3α-hydroxy-cis-bicyclo[3,3,0]octan-7-one

$$IR\ \nu_{cm^{-1}}^{film}: 3450, 2920, 1735, 1595$$

A solution of sodium methylsulfinylmethide, prepared from 65% sodium hydride (1.6 g) and dimethylsulfoxide (21 ml), was cooled to 15°C-20°C and treated with 4-carboxybutyl triphenylphosphonium bromide (7.72 g) under nitrogen. The mixture was stirred for 10 min at 20°C.

To the above ylide solution was added a dimethylsulfoxide solution of 2β-trityloxymethyl-3α-hydroxy-cis-bicyclo[3,3,0]octan-7-one (2.41 g) obtained above at 20°C-25°C. The reaction mixture was stirred for 21 hrs at room temperature and then poured into ice-water. The mixture was washed with diethyl ether and the aqueous layer was acidified with aqueous potassium bisulfate and extracted with ethyl acetate.

The ethyl acetate extract was washed with water, dried and concentrated in vacuo to give the residue, which was then chromatographed on silica gel to yield 2β-trityloxymethyl-3α-hydroxy-7E-(4"-carboxy-butylidene)-cis-bicyclo[3,3,0]octane.

IR $\nu^{film}_{cm^{-1}}$: 2950, 2550, 1720-1700

NMR δ($CDCl_3$): 5.4-4.9(1H, br)

Referential Example 17

A diethyl ether solution of 2β-trityloxy-methyl-3α-hydroxy-7E-(4"-carboxybuthylidene)-cis-bicyclo-[3,3,0]octane (1.9 g) was treated with a diethyl ether solution of diazomethane.

The methyl ester thus obtained was dissolved in pyridine and acetic anhydride (5.2 g) was added. The mixture was stirred for 24 hrs at room temperature, then poured into ice-water, and extracted with ethyl acetate. The extract was dried and concentrated in vacuo to give oily 2β-trityloxymethyl-3α-acetoxy-7E-(4"-methoxycarbonylbutylidene)-cis-bicyclo[3,3,0]octane.

IR $\nu^{film}_{cm^{-1}}$: 2925, 1740-1720, 1060

NMR δ($CDCl_3$): 3.6(3H, s), 2.0(3H, s).

The ester compound (2.8 g) obtained above was dissolved in methyl acetate (90 ml) and cooled with ice-water. To this solution was added concentrated hydrochloric acid (9.0 g). The mixture was stirred for 20 min at the same temperature, and then neutralized with saturated sodium bicarbonate, extracted with ethyl acetate and dried. After evaporation of the solvent, the residue was chromatographed on silica gel to give oily 2β-hydroxymethyl-3α-acetoxy-7E-(4"-methoxycarbonylbutylidene)-cis-bicyclo[3,3,0]octane.

IR $\nu^{film}_{cm^{-1}}$: 3450, 2925, 1730-1710

NMR δ($CDCl_3$): 5.25(1H, br), 4.95(1H, m), 3.7(3H, s) 3,6(2H, d), 2.05(3H, s)

The alcohol compound (0.24 g) obtained above was dissolved in dichloromethane (20 ml) and 0.96 g of pyridinium chlorochromate. The mixture was stirred for 5 hrs at room temperature. To this mixture was added 200 ml of diethyl ether and stirred for further 1 hr at the same temperature. After filtration of the undissolved material, the filtrate was concentrated to give oily 2β-formyl-3α-acetoxy-7E-(4"-methoxycarbonylbutylidene)-cis-bicyclo[3,3,0]octane.

Example 1

A solution of sodium methylsulfinylmethide prepared from 60% sodium hydride (1.44 g) and dimethylsulfoxide (40 ml) was cooled to 15°C-20°C and treated with 4-carboxybutyl triphenylphosphonium bromide (8.0 g) under nitrogen.

The mixture was stirred for 10 min at 20°C, and to this solution was added a dimethylsulfoxide solution of 2β-[8'-hydroxy-3'-acetoxy-trans-1'-nonenyl]-3α-hydroxy-cis-bicyclo[3,3,0]octan-7-one (0.2 g,

Referential Example 14) at 20°C-25°C. The reaction mixture was stirred for 24 hrs at room temperature, and then diluted with water and 1 N aqueous sodium hydroxide.

The aqueous solution was washed with diethyl ether, acidified with 0.5 N aqueous potassium bisulfate and extracted with ethyl acetate. The latter extract was dried and evaporated in vacuo. The residue was dissolved in ethyl acetate and treated with an etheral diazomethane solution. After evaporation of solvent, the residue was chromatographed on silica gel column to give 2β-(8',3'-dihydroxy-trans-1'-nonenyl)-3α-hydroxy-(4"-methoxycarbonylbutylidene)-cis bicyclo[3,3,0] octane (0.15 g).

NMR δ($CDCl_3$): 5.6-5.4 (2H, m), 5.3-5.1 (1H, m) 3.65 (3H, s), 1.2(3H, d)

Example 2.

In the same manner as Example 1, there were obtained the following compounds from the corresponding ketons (Referential example 14 or 15).

2β-(8',3'-dihydroxy-1'-nonynyl)-3α-hydroxy-7-(4"-methoxycarbonylbutylidene)-cis-bicyclo[3,3,0]octane

NMR δ($CDCl_3$): 5.4-5.1(1H, m), 4.5-4.27(1H, m) 4.1-3.7(2H, m), 3.65(3H, s) 1.17(3H, d)

2β-(11',3'α-dihydroxy-trans-1'-dodecenyl)-3α-hydroxy-7-(4"-methoxycarbonylbutylidene)-cis-bicyclo[3,3,0]octane

NMR δ($CDCl_3$): 5.55-5.4(2H, m), 5.4-5.1(1H, m)

3.65(3H, s), 1.17(3H, d)

2β-(11',3'β-dihydroxy-trans-1'-dodecenyl)-3α-hydroxy-7-(4"-methoxycarbonylbutylidene)-cis-bicyclo[3,3,0]octane

NMR δ($CDCl_3$): 5.65-5.45(2H, m), 4.2-3.6(3H, m) 3.68(3H, s), 1.20(3H, d)

2β-(9',3'α-dihydroxy-4',4'-dimethyl-trans-1'-nonenyl)-3α-hydroxy-7-(4"-methoxycarbonylbutylidene)-cis-bicyclo-[3.3.0]octane

NMR δ($CDCl_3$): 5.55-5.45(2H, m), 5.3-5.1(1H, m) 4.03-3.60(4H, m), 3.67(3H, s), 0.90(6H).

2β-(9',3'β-dihydroxy-4',4'-dimethyl-trans-1'-nonenyl)-3α-hydroxy-7-(4"-methoxycarbonylbutylidne)-cis-bicyclo [3,3,0]octane

NMR δ($CDCl_3$): 5.6-5.4(2H, m), 5.23-5.13(1H, m) 4.17-3.57(4H, m), 3.63(3H, s), 0.90(6H)

2β-(10',3'-dihydroxy-trans-1'-decenyl)-3α-hydroxy-7-(4"-methoxycarbonylbutylidene)-cis-bicyclo[3,3,0]octane

NMR δ($CDCl_3$): 5.6-5.4(2H, m), 5.3-5.1(1H, m), 4.2-3.6(4H, m), 3.65(3H, s)

2β-(8',3'α-dihydroxy-trans-1'-octenyl)-3α-hydroxy-7-(4"-methoxycarbonylbutylidene)-cis-bicyclo[3,3,0]octane

NMR δ($CDCl_3$): 5.7-5.3(2H, m), 5.3-5.1(1H, m) 3.66(3H, s).

2β-(9',3'α-dihydroxy-trans-1'-nonenyl)-3α-hydroxy-7-(4"-methoxycarbonylbutylidene)-cis-bicyclo[3,3,0]octane

NMR δ($CDCl_3$): 5.6-5.4(2H, m), 5.35-5.05(1H, m)

3.65(3H, s).

2β-(9',3'β-dihydroxy-4',5'-dimethyl-trans-1'-nonenyl) 3α-hydroxy-7-(4"-methoxycarbonylbutylidene)-cis-bicyclo-[3,3,0]octane

NMR δ($CDCl_3$): 5.6-5.4(2H, m), 5.4-5.1(1H, m) 0.95(3H, d), 0.85(3H, d).

2β-(9',3'α-dihydroxy-4',5'-dimethyl-trans-1'-nonenyl)-3α-hydroxy-7-(4"-methoxycarbonylbutylidene)-cis-bicyclo-[3,3,0]octane

NMR δ($CDCl_3$): 5.7-5.5(2H, m), 5.4-5.1(1H, m) 0.90(3H, d), 0.88(3H, d)

2β-(8',3'α-dihydroxy-5'-methyl-trans-1'-octenyl)-3α-hydroxy-7-(4"-methoxycarbonylbutylidene)-cis-bicyclo-[3,3,0]octane

NMR δ($CDCl_3$): 5.75-5.45(2H, br), 5.45-5.1(1H, m) 4.35-4.0(1H, br), 3.65(3H, s), 1.0-0.83(3H, m)

2β-(8',3'-dihydroxy-octyl)-3α-hydroxy-7-(4"-methoxy-carbonylbutylidene)-cis-bicyclo[3,3,0]octane

NMR δ($CDCl_3$): 5.35-5.05(1H, m), 3.65(3H, s)

2β-(8',3'-dihydroxy-3'-methyl-trans-1'-octenyl)-3α-hydroxy-7-(4"-methoxycarbonylbutylidene)-cis-bicyclo-[3,3,0]octane

NMR δ($CDCl_3$): 5.75-5.4(2H, m), 5.35-5.05(1H, m) 3.60(3H, s), 1.27(3H, s).

Example 3

A methanolic solution (5 ml) of 2β-(8',3'-

dihydroxy-trans-1'-nonenyl)-3α-hydroxy-7-(4"-methoxycarbonylbutylidene)-cis-bicyclo[3,3,0]octane (0.1 g) was treated with 1N sodium hydroxide (3 ml). The mixturae was stirred for 5 hr at room temperature and condensed under reduced pressure. Water and diethyl ether were added and the organic layer was separated off. The aqueous layer was acidified with 0.1 N potassium bisulfate and extracted with ethyl acetate. The ethyl acetate layer was dried and concentrated to yield 2β-(8',3'-dihydroxy-trans-1'-nonenyl)-3α-hydroxy-7-(4"-carboxybutylidene)-cis-bicyclo[3,3,0]octane

NMR δ($CDCl_3$): 5.6-5.4(2H, m), 5.3-5.1(1H, m) 1.2(3H, d).

Example 4

In the same manner as Example 3, there were obtained the following compounds from the corresponding esters (Example 2).

2β-(8',3'-dihydroxy-1'-nonynyl)-3α-hydroxy-7-(4"-carboxybutylidene)-cis-bicyclo[3,3,0]octane

NMR δ($CDCl_3$): 5.4-5.1(1H, m), 4.5-4.2(1H, m) 4.1-3.7(2H, m), 1.17(3H, d)

2β-(11',3'α-dihydroxy-trans-1'-dodecenyl)-3α-hydroxy-7-(4"-carboxybutylidene)-cis-bicyclo[3,3,0]octane

NMR δ($CDCl_3$): 5.6-5.4(2H, m), 5.4-5.1(1H, m), 1.17(3H, d)

2β-(11',3'β-dihydroxy-trans-1'-dodecenyl)3α-hydroxy-7-(4"-carboxybutylidene)-cis-bicyclo[3,3,0]octane

NMR δ($CDCl_3$): 5.7-5.4(2H, m), 5.4-5.1(1H, m) 1.20(3H, d)

2β-(9',3'α-dihydroxy-4',4'-dimethyl-trans-1'-nonenyl)-3α-hydroxy-7-(4"-carboxybutylidene)-cis-bicyclo[3,3,0]-octane

NMR δ($CDCl_3$): 5.48(2H, m), 5.22(1H, m), 4.70(4H, br), 4.10-3.53(4H, m) 0.88(6H)

2β-(9',3'β-dihydroxy-4',4'-dimethyl-trans-1'-nonenyl)-3α-hydroxy-7-(4"-carboxybutylidene)-cis-bicyclo[3,3,0]-octane

NMR δ($CDCl_3$): 5.57(2H, m), 5.17(5H, br) 4.23-3.6(4H, m), 0.90(6H)

2β-(10',3'-dihydroxy-trans-1'-decenyl)-3α-hydroxy-7-(4"-carboxybutylidene)-cis-bicyclo[3,3,0]octane

NMR δ($CDCl_3$): 5.7-5.4(2H, m), 5.35-5.05(1H, m)

2β(9',3'α-dihydroxy-trans-1'-nonenyl)-3α-hydroxy-7-(4"-carboxybutylidene)-cis-bicyclo[3,3,0]octane

NMR δ ($CDCl_3$): 5.6-5.4(2H, m), 5.35-5.05(1H, m)

2β-(9',3'α-dihydroxy-4',5'-dimethyl-trans-1'-nonenyl)-3α-hydroxy-7-(4"-carboxybutylidene)-cis-bicyclo[3,3,0]-octane

NMR δ($CDCl_3$): 5.7-5.5(2H, m), 5.4-5.1(1H, m), 0.9(3H, d), 0.88(3H, d)

2β-(9',3'β-dihydroxy-4',5'-dimethyl-trans-1'-nonenyl)-3α-hydroxy-7-(4"-carboxybutylidene)-cis-bicyclo[3,3,0]-octane

NMR δ($CDCl_3$): 5.6-5.4(2H, m), 5.4-5.1(1H, m) 0.95(3H, d), 0.85(3H, d)

2β-(8',3'α-hydroxy-5'-methyl-trans-1'-octenyl)-3α-hydroxy-7-(4"-carboxybutylidene)-cis-bicyclo[3,3,0]octane

NMR δ($CDCl_3$): 5.7-5.45(2H, m), 5.45-5.1(1H, m) 4.35-4.0(1H, br)

2β-(8',3'-dihydroxy-octyl)-3α-hydroxy-7-(4"-carboxybutylidene)-cis-bicyclo[3,3,0]octane

NMR δ($CDCl_3$): 5.35-5.10(1H, m), 3.8-3.53(2H, m)

2β-(8',3'-dihydroxy-3'-methyl-trans-1'-octenyl)-3α-hydroxy-7-(4"-carboxybutylidene)-cis-bicyclo-[3,3,0]-octane

NMR δ($CDCl_3$): 6.0-5.7(4H, br). 5.6-5.4(2H, m) 5.3-5.0(1H, m). 3.7-3.4(2H, m) 1.23(3H, s)

Example 5

Following the procedures of Example 3, 2β-(8',-3'α-dihydroxy-trans-1'-octenyl)-3α-hydroxy-7-(4"-methoxycarbonylbutylidene)-cis-bicyclo[3,3,0]octane (0.1 g, Example 2) was hydrolized to give an oily residue (0.08 g). The residue was chromatographed on silicagel using ethyl acetate as an eluent to afford a less polar 2β-(8',-3'α-dihydroxy-trans-1'-octenyl)-3α-hydroxy-7Z-(4"-carboxy-butylidene)-cis-bicyclo[3,3,0]octane (10 mg) and a more polar 2β-(8',3'α-dihydroxy-trans-1'-octenyl)-3α-hydroxy-7E-(4"-carboxybutylidene)-cis-bicyclo[3,3,0]octane (25 mg).

NMR $\delta(CDCl_3)$ 7Z: 5.7-5.3(2H, m), 5.3-5.1(1H, m)
4.2-3.9(2H, m), 3.9-3.5(2H, m)
7E: 5.7-5.3(2H, m), 5.3-5.1(1H, m)
4.3-3.9(2H, m), 3.85-3.5(2H, m)

Example 6

Following the procedure of Example 1, 2β-(8'-hydroxy-3α-acetoxy-trans-1'-octenyl)-3α-hydroxy-cis-bicyclo-[3,3,0]octan-7-one (0.3 g, Referential example 14) was treated with the ylide prepared from sodium hydride (60%, 1.18 g), dimethyl-sulfoxide and 4-carboxybutyl triphenylphosphonium bromide (7.1 g) to yield crude carboxylic compound (0.69 g).

This crude compound was chromotographed on silica gel using ethyl acetate to afford less polar 2β-(8',3'α-dihydroxy-trans-1'-octenyl)-3α-hydroxy-7Z-(4"-carboxybutylidene)-cis-bicyclo[3,3,0]octane (50 mg) and more polar 2β-(8',3'α-dihydroxy-trans-1'-octenyl)-3α-hydroxy-7E-(4"-carboxybutylidene)-cis-bicyclo[3,3,0]-octane (120 mg).

Spectral data of these isomers were coincided with those obtained in Example 5.

Example 7

A tetrahydrofuran (10 ml) solution of 2β-formyl-3α-acetoxy-7E-(4"-methoxycarbonylbutylidene)-cis-bicyclo-[3,3,0]octane (0.64 g, obtained in Referential Example 17) was added at 34°C under nitrogen to a tetrahydrofuran solution (35 ml) of the ylide prepared with

dimethyl 2-oxo-7-hydroxy-heptylphosphonate (1.89 g) and sodium hydride (50%, 0.24 g). The mixture was stirred for 30 min at 30°C and then poured into water and extracted with ethyl acetate. The extract was washed with water, dried and concentrated in vacuo. The residue was chromatographed on silica gel to give 2β-[8'-hydroxy-3'-oxo-trans-1'-octenyl]-3α-acetoxy-7E-(4"-methoxycarbonylbutylidene)-cis-bicyclo[3,3,0]octane (0.65 g).

IR $\nu_{cm^{-1}}^{film}$: 2900, 1735, 1680, 1620

NMR $\delta(CDCl_3)$: 6.67(1H, d, d,), 6.02(1H, d), 5.03-4.70(1H, m), 3.67(3H, s), 2.0(3H, s).

In the same manner as aove, there were obtained the following compounds by changing the Wittig reagents (Referential example 2).

2β-(8'-hydroxy-3'-oxo-trans-1'-nonenyl)-3α-acetoxy-7E-(4"-methoxycarbonylbutylidene)-cis-bicyclo[3,3,0]octane

NMR $\delta(CDCl_3)$: 6.60(1H, d, d) 5.95(1H, d) 5.33-5.1(1H, m) 5.0-4.7(1H, m) 3.63(3H, s), 1.98(3H, s)

2β-[4'-(4"-tetrahyropyranyloxybutoxy)-3'-oxo-trans-1'-butenyl]-3α-acetoxy-7E-(4"-methoxycarbonylbutylidene)-cis-bicyclo[3,3,0]octane

NMR $\delta(CDCl_3)$: 6.83(1H, d, d), 6.25(1H, d) 5.4-5.3(1H, br) 3.67(3H, s) 2.00(3H, s)

2β-[4'-(4"β-tetrahydropyranyloxy-cyclohexyl)-3'-oxo-trans-1'-butenyl]-3α-acetoxy-7E-(4"-methoxycarbonyl-butylidene)-cis-bicyclo[3,3,0]octane

NMR δ($CDCl_3$): 6.58(1H, d, d), 5.97(1H, d)
3.66(3H, s), 1.98(3H, s)

2β-[4'-(4"α-tetrahydropyranyloxy-cyclohexyl)-3'-oxo-trans-1'-butenyl]-3α-acetoxy-7E-(4"-methoxycarbonyl-butylidene)-cis-bicyclo[3,3,0]octane

NMR δ($CDCl_3$): 6.57(1H, d, d), 5.90(1H, d)
3.67(3H, s) 1.97(3H, s)

Example 8

A methanolic solution (100 ml) of 2β-[8'-hydroxy-3'-oxo-trans-1'-octenyl]-3α-acetoxy-7E-(4"-methoxycarbonylbutylidene)-cis-bicyclo[3,3,0]octane (300 mg) obtained above was treated with a chilled methanolic solution of sodium borohydride (100 mg) at 5°C, and stirreed for 2 hrs at the same temperature. The excess reducing agent was quenched with acetone, and the mixture was concentrated in vacuo. To the residue was added aqueous ammonium chloride and the mixture was extracted with ethyl acetate. The extract was washed with water, dried and concentrated in vacuo. The residue was chromatographed on silica gel to give less polar 2β-(8',3'β-dihydroxy-trans-1'-octenyl)-3α-hydroxy-7E-(4"-methoxycarbonylbutylidene)-cis-bicyclo[3,3,0]octane (75 mg) and more polar 2β-(8',3'α-dihydroxy-trans-1'-octenyl)-3α-hydroxy-7E-(4"-methoxycarbonylbutylidene)-cis-bicyclo-

[3,3,0]octane (180 mg).

NMR $\delta(CDCl_3)$ 3'β 5.57-5.43(2H, m), 5,37-5.13(1H, m) 3.70(3H, s), 2.03(3H, s).

3'α 5.6-5.43(2H, m), 5.37-5.1(1H, m) 3.63(3H, s), 2.0(3H, s)

In the same manner as above example, there were obtained the following compounds from the corresponding ketones (Example 7).

2β-(8',3'β-dihydroxy-trans-1'-nonenyl)-3α-acetoxy-7E-(4"-methoxycarbonylbutylidene)-cis-bicyclo[3,3,0]octane

NMR $\delta(CDCl_3)$: 5.57-5.43(2H, m), 5.37-5.16(1H, m), 3.6(3H, s), 2.00(3H, s).

2β-(8',3'α-dihydroxy-trans-1'-nonenyl)-3α-acetoxy-7E-(4"-methoxycarbonylbutylidene)-cis-bicyclo[3,3,0]octane

NMR $\delta(CDCl_3)$: 5.57-5.43(2H, m), 5.37-5.13(1H, m) 3.67(3H, s), 2.0(3H, s)

2β-[4'-(4"-tetrahydropyranyloxybutoxy)-3'-hydroxy-trans-1'-butenyl]-3α-acetoxy-7E-(4"-methoxycarbonyl-butylidene)-cis-bicyclo[3,3,0]octane

NMR $\delta(CDCl_3)$: 5.6-5.4(2H, m), 5.4-5.0(1H, m) 3.63(3H, s), 1.98(3H, s)

2β-[4'-(4"β-tetrahydropyranyloxy-cyclohexyl)-3'α-hydroxy-trans-1'-butenyl]-3α-acetoxy-7E-(4"-methoxy-carbonylbutylidene)-cis-bicyclo[3,3,0]octane

NMR $\delta(CDCl_3)$: 5.57-5.37(2H, m), 5.33-5.07(1H, m) 3.67(3H, s), 2.0(3H, s)

2β-[4'-(4"β-tetrahydropyranyloxy-cyclohexyl)-3'β-hydroxy-trans-1'-butenyl]-3α-acetoxy-7E-(4"-methoxy-carbonylbutylidene)-cis-bicyclo[3,3,0]octane

NMR δ($CDCl_3$): 5.73-5.4(2H, m). 5.4-5.1(1H, m) 3.67(3H, s), 1.98(3H, s)

2β-[4'-(4"α-tetrahydropyranyloxy-cyclohexyl)-3'-hydroxy-trans-1'-butenyl]-3α-acetoxy-7E-(4"-methoxy-carbonylbutylidene)-cis-bicyclo[3,3,0]octane

NMR δ($CDCl_3$): 5.6-5.43(2H, m) 5.4-5.07(1H, m) 3.67(3H, s), 2.0(3H, s)

Example 9

A mixture of 2β-(8',3'α-dihydroxy-trans-1'-octenyl)-3α-acetoxy-7E-(4"-methoxycarbonylbutylidene)-cis-bicyclo[3,3,0]octane (30 mg), sodium hydroxide (80 mg), 0.6 g of water and 0.6 ml of methanol was stirred for 70 min at room temperature. The mixture was diluted with water and washed with diehtyl ether. The aqueous layer was acidified with 10% hydrochloric acid and extracted with ethyl acetate. The extract was washed with water, dried and concentrated in vacuo to give 2β-(8',3'α-dihydroxy-trans-1'-octenyl)-3α-hydroxy-7E-(4"-carboxybutylidene)-cis-bicyclo[3,3,0]octane (40 mg).

Spectral data of this compound was coincided with those obtained in Example 5.

In the same manner as above exmaple, there were obtained the following compounds from the corresponding esters (Example 8).

2β-(8',3'β-dihydroxy-trans-1'-octenyl)-3α-hydroxy-7E-(4"-carboxybutylidene)-cis-bicyclo[3,3,0]octane

NMR δ($CDCl_3$): 5.63-5.43(2H, m), 5.3-5.10(1H, m) 3.6(2H, m)

2β-(8',3'α-dihydroxy-trans-1'-nonenyl)-3α-hydroxy-7E-(4"-carboxybutylidene)-cis-bicyclo[3,3,0]octane

NMR δ($CDCl_3$): 5.65-5.4(2H, m), 5.3-5.0(1H, m) 1.17(3H, d)

2β-(8',3'β-dihydroxy-trans-1'-nonenyl)-3α-hydroxy-7E-(4"-carboxybutylidene)-cis-bicyclo[3,3,0]octane

NMR δ($CDCl_3$): 5.6-5.4(2H, m), 5.3-5.0(1H, m) 1.17(3H, d)

Example 10

A mixture of 2β-[4'-(4"-tetrahydropyranyoxy-butoxy)-3'-hydroxy-trans-1'-butenyl]-3α-acetoxy-7E-(4"-methoxycarbonylbutylidene)-cis-bicyclo[3,3,0]octane (60 mg), pyridinium p-toluensulfonic acid (20 mg) and ethanol (5 ml) was heated for 2 hr at 60°. The solvent was evaporated under reduced pressure and the residue was chromatogaraphed on silica gel to afford 2β-[4'-(4"-hydroxybutoxy)-3'-hydroxy-trans-1'-butenyl]-3α-acetoxy-7E-(4"-methoxycarbonylbutylidene)-cis-bicyclo[3,3,0]octane (30 mg).

NMR δ($CDCl_3$): 5.6-5.4(2H, m), 5.4-5.0(1H, m) 3.67(3H, s)

The alcohol obtained above was dissolved in methanol (5 ml) and treated with 1N sodium hydroxide (5 ml). The mixture was stirred for 2 hr and then condensed in vacuo. The residue was diluted with water and washed with diethyl ether. The aqueous layer was acidified with 0.5 N potassium bisulfate and extracted with ethyl acetate. The extract was dried and concentrated in vacuo to give 2β-[4'-(4"-hydroxybutoxy)-3'-hydroxy-trans-1'-butenyl]-3α-hydroxy-7E-(4"-carboxybutylidene)-cis-bicyclo[3,3,0]octane (14 mg).

NMR δ($CDCl_3$): 5.63-5.37(2H, m), 5.37-5.0(1H, m)

In the same manner as the Example above, there were obtained the following compounds.

2β-[4'-(4"β-hydroxy-cyclohexyl)-3'α-hydroxy-trans-1'-butenyl]-3α-hydroxy-7E-(4"-carboxybutylidene)-cis-bicyclo[3,3,0]octane

NMR δ($CDCl_3$): 5.7-5.4(2H, m), 5.4-5.1(1H, m)

2β-[4'-(4"β-hydroxy-cyclohexyl)-3'β-hydroxy-trans-1'-butenyl]-3α-hydroxy-7E-(4"-carboxybutylidene)-cis-bicyclo-[3,3,0]octane

NMR δ($CDCl_3$): 5.6-5.4(2H, m), 5.35-5.05(1H, m)

2β-[4'-(4"α-hydroxy-cyclohexyl)-3'-hydroxy-trans-1'-butenyl]-3α-hydroxy-7E-(4"-carboxybutylidene)-cis-bicyclo[3,3,0]octane

NMR δ($CDCl_3$): 5.6-5.43(2H, m), 5.4-5.05(1H, m)

Example 11

Acetic anhydride (5 ml) was added to a pyridine solution (5 ml) of 2β-[3',8'-dihydroxy-trans-1'-nonenyl]-3α-hydroxy-7-(4"-methoxycarbonylbutylidene)-cis-bicyclo[3,3,0]octane (50 mg, obtianed in Example 1).

The mixture was stirred for 17 hrs at room temperature and then diluted with water and extracted with ethyl acetate. The extract was washed with water and concentrated in vacuo to give oily 2β-(3',8'-diacetoxy-trans-1'-nonenyl)-3α-acetoxy-7-(4"-methoxycarbonylbutylidene)-cis-bicyclo[3,3,0]octane (60 mg).

NMR δ($CDCl_3$): 5.6-5.35(2H, m), 5.35-5.0(2H, m), 4.95-4.65(1H, m), 3.63(3H, s), 2.00(3H, s), 1.98(3H, s), 1.95(3H, s)

In the same manner as above, there were obtained the following compounds from the corresponding alcohols (Example 2).

2β-(3',8'-diacetoxy-trans-1'-octenyl)-3α-acetoxy-7-(4"-methoxycarbonylbutylidene)-cis-bicyclo[3,3,0]octane

NMR δ($CDCl_3$): 5.7-5.3(2H, m), 5.3-5.0(2H, m), 4.97-4.7(1H, m), 3.60(3H, s), 1.97(9H, s)

2β-(3',8'-diacetoxy-octyl)-3α-acetoxy-7-(4"-methoxycarbonylbutylidene)-cis-bicyclo[3,3,0]octane

NMR (δ$CDCl_3$): 5.35-5.07(1H, m), 4.95-4.5(2H, m) 3.61(3H, s), 1.98(9H, s)

Example 12

To a mixture of 2β-[8',3'α-dihydroxy-trans-1'-octenyl]-3α-hydroxy-7-(4"-carboxy-butylidene)-cis-bicyclo[3,3,0]octane (30 mg, obtained in Example 2) and pyridine (2 ml) was added acetic anhydride (2 ml). The mixture was stirred for 24 hrs at room temperature, then

poured into water, and extracted with ethyl acetate. The extract was washed with water, dried and concentrated in vacuo to give 2β-[8',3'α-diacetoxy-trans-1'-octenyl]-3α-acetoxy-7-(4"-carboxy-butylidene)-cis-bicyclo[3,3,0]octane

IR $\nu_{cm^{-1}}^{film}$: 2600,1730-1700

NMR $\delta(CDCl_3)$: 5.7-5.4(2H, m), 5.3-5.0(2H, m), 4.97-4.7(1H, m), 2.0(9H, s)

WHAT IS CLAIMED IS:

1. A compound of the general formula:

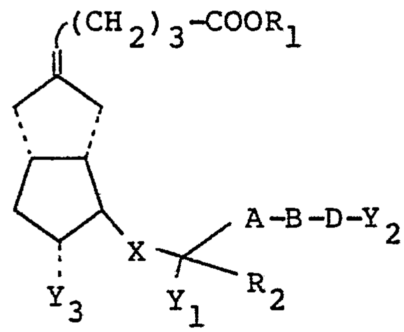

[I]

wherein

$R_1$ is a hydrogen atom or a $C_1$-$C_4$ alkyl group,

$R_2$ is a hydrogen atom or a $C_1$-$C_5$ alkyl group,

A is a $C_1$-$C_{10}$ alkylene group,

B is a direct bond, a cycloalkylene, or a group of either one of the formulae: $-O-$, $-CH{=}CH-$, $-C{\equiv}C-$, or 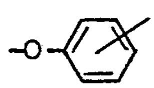 (o,m,p- phenylene or phenoxylene), 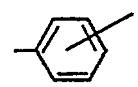

D is a $C_1$-$C_5$ alkylene or a cycloalkylene group,

X is an ethylene, a vinylene or an ethynylene group,

$Y_1$, $Y_2$ and $Y_3$ are each independently a free or a protected hydroxy group or a pharmaceutically acceptable salt of such a compound wherein $R_1$ is a hydrogen atom.

2. A compound as claimed in claim 1, wherein B is a direct bond.

3. A compound as claimed in claim 1, wherein X is a vinylene group.

4. A compound as claimed in claim 2, wherein X is a vinylene group.

5. A compound as claimed in claim 1, wherein $Y_1$,

$Y_2$ and $Y_3$ are each independently a free or a protected hydroxy group selected from the group consisting of trisubstituted silyl group, 5 or 6 membered heterocyclic group, a lower alkyl group, a lower alkanoyl group or an aroyl group.

6. A compound as claimed in claim 5, wherein B is a direct bond.

7. A compound as claimed in claim 5, wherein X is a vinylene group.

8. A compound as claimed in claim 5, wherein B is a direct bond and X is a vinylene group.

9. A compound as claimed in claim 5, wherein $Y_1$, $Y_2$ and $Y_3$ are each a free hydroxy group.

10. A compound as claimed in claim 9, wherein B is a direct bond.

11. A compound as claimed in claim 9, wherein X is a vinylene group.

12. A compound as claimed in claim 9, wherein B is a direct bond and X is a vinylene group.

13. A compound as claimed in claim 5, wherein $Y_1$, $Y_2$ and $Y_3$ are each independently a lower alkanoyl group.

14. A compound as claimed in claim 13, wherein B is a direct bond.

15. A compound as claimed in claim 13, wherein X is a vinylene group.

16. A compound as claimed in claim 13, wherein B is a direct bond and X is a vinylene group.

17. A compound as claimed in any one of claims 1 to 16, wherein $Y_1$ is in α-configuration.

18. A compound as claimed in any one of claims 1 to 16, wherein 7-position is in E-form.

19. 2β-(3'α,8'-dihydroxy-trans-1'-octenyl)-3α-hydroxy-7E-(4"-carboxybutylidene)-cis-bicyclo [3,3,0] octane, and its stereoisomers (3'β and/or 7Z)

20. 2β-(3'α,8'-dihydroxy-octyl)-3α-hydroxy-7E-(4"-carboxybutylidene)-cis-bicyclo [3,3,0] octane, and its stereoisomers (3'β, and/or 7Z)

21. 2β-(3'α,8'-dihydroxy-3'β-methyl-trans-1'-octenyl)-3α-hydroxy-7E-(4"-carboxybutylidene)-cis-bicyclo [3,3,0] octane, and its stereoisomers (3'β and/or 7Z)

22. 2β-(3'α,8'-dihydroxy-5'-methyl-trans-1'-octenyl)-3α-hydroxy-7E-(4"-carboxybutylidene)-cis-bicyclo [3,3,0] octane, and its stereoisomers (3'β and/or 7Z)

23. 2β-(3'α,8'-dihydroxy-trans-1'-nonenyl)-3α-hydroxy-7E-(4"-carboxybutylidene)-cis-bicyclo [3,3,0] octane, and its stereoisomers (3'β and/or 7Z)

24. 2β-(3'α,8'-dihydroxy-1'-nonynyl)-3α-hydroxy-7E-(4"-carboxybutylidene)-cis-bicyclo [3,3,0] octane, and its stereoisomers (3'β and/or 7Z)

25. 2β-(3'α,9'-dihydroxy-1'-nonenyl)-3α-hydroxy-7E-(4"-carboxybutylidene)-cis-bicyclo [3,3,0] octane, and its stereoisomers (3'β and/or 7Z)

26. 2β-(3'α,10'-dihydroxy-trans-1'-decenyl)-3α-hydroxy-7E-(4"-carboxybutylidene)-cis-bicyclo [3,3,0] octane, and its stereoisomers (3'β and/or 7Z)

27. 2β-(3'α,9'-dihydroxy-4',4'-dimethyl-trans-1'-nonenyl)-3α-hydroxy-7E-(4"-carboxybutylidene)-cis-bicyclo [3,3,0] octane, and its stereoisomers (3'β and/or 7Z)

28. 2β-(3'α,9'-dihydroxy-4',5'-dimethyl-trans-1'-nonenyl)-3α-hydroxy-7E-(4"-carboxybutylidene)-cis-bicyclo [3,3,0] octane, and its stereoisomers (3'β and/or 7Z)

29. 2β-(3'α,11'-dihydroxy-trans-1'-dodecenyl)-3α-hydroxy-7E-(4"-carboxybutylidene)-cis-bicyclo [3,3,0] octane, and its steroisomers (3'β and/or 7Z)

30. 2β-[3'α-hydroxy-4'-(4"-hydroxybutoxy)-trans-1'-butenyl]-3α-hydroxy-7E-(4"-carboxybutylidene)-cis-bicyclo [3,3,0] octane, and its stereosiomers (3'β and/or 7Z)

31. 2β-[3'α-hydroxy-4'-(4"β-hydroxy-cyclohexyl)-trans-1'-butenyl]-3α-hydroxy-7E-(4"-carboxybutylidene)-cis-bicyclo [3,3,0] octane, and its stereoisomers (3'β, 4"α and/or 7Z)

32. 2β-(3'α,8'-diacetoxy-trans-1'-octenyl)-3α-acetoxy-7E-(4"-methoxycarbonylbutylidene)-cis-bicyclo [3,3,0] octane, and its stereoisomers (3'β and/or 7Z)

33. 2β-(3'α,8'-diacetoxy-trans-1'-octenyl)-3α-acetoxy-7E-(4"-carboxybutylidene)-cis-bicyclo [3,3,0]

octane, and its stereoisomers (3'β and/or 7Z)

34. A process for producing a compound of the formula:

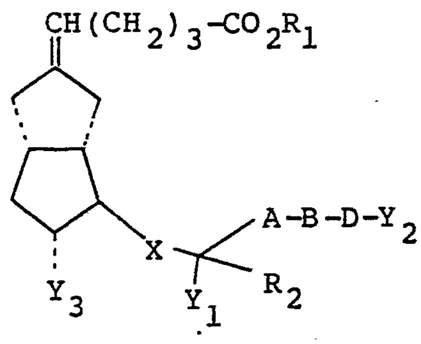

wherein $R_1$, $R_2$, A, B, D, X, $Y_1$, $Y_2$ and $Y_3$ are each as defined in claim 1, and its pharmaceutically acceptable salt, which comprises reacting a compound of the formula:

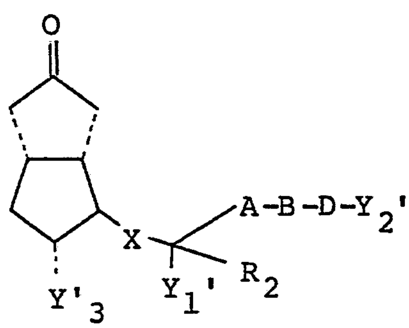

wherein $R_2$, A, B, D and X are each as defined above, and $Y_1'$, $Y_2'$ and $Y_3'$ are independently of each other a free or protected hydroxy group with a compound of the formula:

$$Ph_3P{=}CH(CH_2)_3COOM$$

wherein M is a hydrogen atom or an alkali metal and Ph is a phenyl group,

and if necessary, subsequently one or more of the following reactions are carried out at any appropriate step in any desired sequence:

(1) separation of a mixture of isomers,

(2) deprotection of a protected hydroxy group,

(3) protection of a free hydroxy group

(4) esterification of carboxy group

(5) hydrolysis of an esterified carboxy group

(6) conversion of carboxy group into a salt

35. A process for producing a compound of the formula:

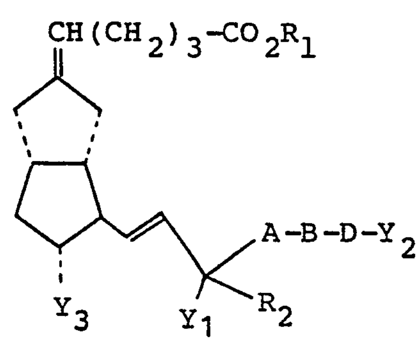

wherein $R_1$, $R_2$, A, B, D, $Y_1$, $Y_2$ and $Y_3$ are each as defined in claim 1, and its pharmaceutically acceptable salt, which comprises reacting a compound of the formula:

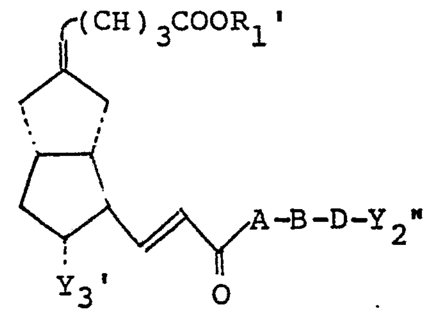

wherein A, B and D are each as defined in claim 1, $R_1'$ is a $C_1$-$C_4$ alkyl group, $Y_3'$ is a free or protected hydroxy group, and $Y_2''$ is a free or protected hydroxy group which may be replaced with a hydrogen atom by acidic conditions, with a reducing agent or an organometallic compound of the formula

$$M'-R_2'$$

wherein $R_2'$ is a $C_1$-$C_5$ alkyl group and M' is a lithium atom or Mg halo (halo is a halogen atom), and if necessary, subsequently one or more of the following reactions are carried out at any appropriate step in any desired sequence:

(1) separation of a mixture of isomers

(2) deprotection of protected hydroxy group

(3) protection of a free hydroxy group

(4) hydrolysis of an esterified carboxy group

(5) conversion of carboxy group into a salt

36. A process for producing a compound of the formula:

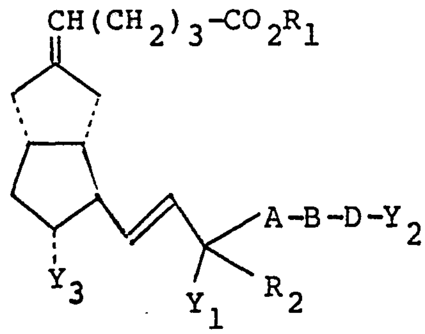

wherein $R_1$, $R_2$, A, B, D, $Y_1$, $Y_2$ and $Y_3$ are each as defined in claim 1, and its pharmaceutically acceptable salt, which comprises reacting a compound of the formula:

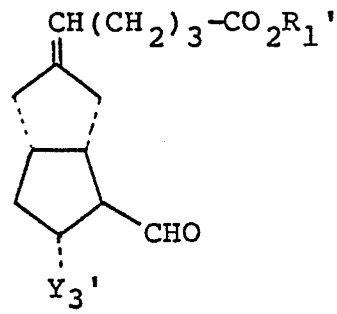

wherein $Y_3'$ is a free or protected hydroxy group and $R'_1$ is a $C_1$-$C_4$ alkyl group, with a compound of the formula

$$(CH_3O)_2\overset{O}{\overset{\uparrow}{P}} = CH-\underset{O}{\underset{\|}{C}}-A-B-D-Y_2''$$

wherein A, B and D are each as defined above, and $Y_2''$ is a free or protected hydroxy group which may be replaced with a hydrogen atom by acidic conditions and further reacting the carbonyl group of the resulting compound with a reducing agent or an organometallic compound of the formula

$M'-R_2'$

wherein $R_2'$ is a $C_1$-$C_5$ alkyl group and M' is a lithium atom or Mg halo (halo is a halogen atom), and if necessary subsequently one or more of the following reactions are carried out at any appropriate step in any desired sequence:

(1) separation of a mixture of isomers

(2) deprotection of a protected hydroxy group

(3) protection of a free hydroxy group

(4) hydrolysis of an esterified carboxy group.

(5) conversion of carboxy group into a salt

37. A pharmaceutical composition which comprises as an active ingredient an effective amount of at least one of the compounds of claim 1, and at least one pharmaceutically acceptable inert carrier or diluent.

38. Use of a compound as claimed in claim 1 as an antiulcer drug.

39. Use of a compound as claimed in claim 1 as an antithrombosis drug.

European Patent Office

0084408

Application number

# EUROPEAN SEARCH REPORT

EP 83 30 0030

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | GB-A-2 013 661 (CARLO ERBA)<br>*The whole document*<br>--- | 1-39 | C 07 C 59/46<br>C 07 C 69/732<br>C 07 C 177/00<br>A 61 K 31/215<br>A 61 K 31/19<br>A 61 K 31/557//<br>C 07 D 317/72 |
| Y | US-A-4 140 712 (M.HAYASHI)<br>*The whole document*<br>----- | 1-39 | |

| TECHNICAL FIELDS SEARCHED (Int. Cl. 3) |
|---|
| C 07 C 59/00<br>C 07 C 69/00<br>C 07 C 177/00 |

The present search report has been drawn up for all claims

| Place of search | Date of completion of the search | Examiner |
|---|---|---|
| THE HAGUE | 11-04-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82